(19) Europäisches Patentamt
European Patent Office
Office européen des brevets

(11) EP 1 406 582 B1

(12) **EUROPEAN PATENT SPECIFICATION**

(45) Date of publication and mention of the grant of the patent: **25.10.2006 Bulletin 2006/43**

(21) Application number: **02747408.9**

(22) Date of filing: **19.06.2002**

(51) Int Cl.:
*A61K 8/37* (2006.01) *A61Q 17/04* (2006.01)
*C07C 69/734* (2006.01)

(86) International application number: **PCT/EP2002/006743**

(87) International publication number: **WO 2003/007906 (30.01.2003 Gazette 2003/05)**

# (54) PHOTOSTABLE ORGANIC SUNSCREEN COMPOUNDS WITH ANTIOXIDANT PROPERTIES AND COMPOSITIONS OBTAINED THEREFROM

PHOTOSTABILE ORGANISCHE SONNENSCHUTZMITTEL MIT ANTIOXIDATIVEN EIGENSCHAFTEN UND ZUSAMMENSETZUNGEN DAMIT

COMPOSES ECRANS SOLAIRES ORGANIQUES PHOTOSTABLES POSSEDANT DES PROPRIETES ANTIOXYDANTES ET COMPOSITIONS OBTENUES A PARTIR DE CES COMPOSES

(84) Designated Contracting States:
**AT BE CH CY DE DK ES FI FR GB GR IE IT LI LU MC NL PT SE TR**

(30) Priority: **16.07.2001 US 904904**
**20.12.2001 US 22343**

(43) Date of publication of application: **14.04.2004 Bulletin 2004/16**

(73) Proprietor: **Merck Patent GmbH**
**64293 Darmstadt (DE)**

(72) Inventor: **CHAUDHURI, Ratan, K.**
**Lincoln Park, NJ 07035 (US)**

(56) References cited:

| | |
|---|---|
| **EP-A- 0 631 177** | **DE-A- 2 816 819** |
| **US-A- 3 278 448** | **US-A- 3 470 233** |
| **US-A- 3 928 324** | **US-A- 5 175 340** |
| **US-A- 5 830 441** | |

- **KNOEVENAGEL, E. ET AL.: CHEM. BER., vol. 37, 1904, pages 4476-4482, XP002218455**
- **SOHDA T ET AL: "Antiulcer activity of 5-benzylthiazolidine-2,4-dione derivatives" CHEMICAL AND PHARMACEUTICAL BULLETIN, PHARMACEUTICAL SOCIETY OF JAPAN. TOKYO, JP, vol. 31, no. 2, February 1983 (1983-02), pages 560-569, XP002193484 ISSN: 0009-2363**
- **CHO, H. ET AL.: J. MED. CHEM., vol. 34, 1991, pages 1503-1506, XP001109032**
- **WRIGHT M E ET AL: "ORGANIC NLO POLYMERS 2. A STUDY OF MAIN-CHAIN AND GUEST-HOST KAPPA(2) NLO POLYMERS: NLO-PHORE STRUCTURE VERSUS POLING" MACROMOLECULES, AMERICAN CHEMICAL SOCIETY. EASTON, US, vol. 27, no. 11, 23 May 1994 (1994-05-23), pages 3009-3015, XP000445674 ISSN: 0024-9297**
- **DATABASE WPI Section Ch, Week 198908 Derwent Publications Ltd., London, GB; Class D21, AN 1989-059208 XP002218456 & JP 01 013017 A (POLA KASEI KOGYO KK), 17 January 1989 (1989-01-17)**
- **GAZIT A ET AL: "TYRPHOSTINS I: SYNTHESIS AND BIOLOGICAL ACTIVITY OF PROTEIN TYROSINE KINASE INHIBITORS" JOURNAL OF MEDICINAL CHEMISTRY, AMERICAN CHEMICAL SOCIETY. WASHINGTON, US, vol. 32, no. 19, 1989, pages 2344-2352, XP002048362 ISSN: 0022-2623**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 406 582 B1

## Description

Background of the invention

Topical sunscreen compositions are commonly used during outdoor work or leisure as a means for providing protection of exposed skin against acute and chronic adverse effects of solar radiation such as sunburn, cancer and photo-aging. Many effective sunscreen preparations are sold commercially or are described in cosmetic or pharmaceutical literature. In general sunscreen preparations are formulated as creams, lotions or oils containing as the active agent an ultra violet radiation absorbing chemical compound. The sunscreen functions by blocking passage of ultra violet radiation thereby preventing its penetration into the skin.

According to Zecchino et al. (US 5,008,100), sunscreen agents may be characterized in the order of decreasing effectiveness as either highly chromophoric (monomeric organic compounds and inorganic compounds such as titanium dioxide) and minimally chromophoric (polymeric organic solids).

Organic sunscreens are classified into UV-A filters, UV-B filters or broad spectrum filters (UV-A and UV-B functionality in a single molecule) depending on the type of radiation they absorb. UV-A sunscreens absorb radiation in the 320 to 400 nm regions of the ultra violet spectrum and UV-B sunscreens absorb radiation in the 290 to 320 nm regions of the ultra violet spectrum. Broad band sunscreens (UV-A and UV-B functionality) absorb radiation in the 290 to 400 nm region of the ultra violet spectrum and have two maximums, one in the UV-B region and the other in the UV-A region.

Representative references related to UV sunscreens are: US Patent No. 3,278,448, which discloses cinnamic acid derivatives such as 4-hydroxy, 3-5-ditertbutyl-alphacarbethoxy-cinnamic acid ether ester in column 2, line 20; US Patent No. 3,538,226, which describes cinnamic acid alkyl ester derivatives at column 1, lines 15-31 and column 2, lines 1-12 and column 3, lines 30-55 and 60; US Patent No. 5,175,340, which describes cinnamic acid alkyl esters having hydroxy radicals and alkoxy radicals on the phenyl ring, and US Patent No. 5,830,441, which describes UV absorbents containing a cyano or cinnamyl moiety by the generic formula at col. 2, lines 1-21. Other references which disclose cinnamide compounds include U.S. Patent Nos. 5,601,811, 4,335,054, 5,124,354, 5,294,643 and 5,514,711.

Unfortunately, some of the highly chromophoric monomeric organic compounds employed in sunscreen compositions are not photostable and the protection from sun damage is lost. In addition to lack of photostability of many organic sunscreens, they do not possess an antioxidant property which is essential for protecting skin or hair.

The ideal sunscreen formulation should be nontoxic and non-irritating to the skin tissue and be capable of convenient application in a uniform continuous film. The product should be chemically and physically stable so as to provide an acceptable shelf life upon storage. It is particularly desirable that the preparation should retain its protective effect over a prolonged period after application. Thus, the active agent when present on the skin must be resistant to chemical and/or photo degradation.

Techniques for stabilizing UV absorbent compositions are known. Representative disclosures in this area include U.S. Patent Nos. 5,567,418, 5,538,716, 5,951,968 and 5,670,140.

It is desirable to provide the antioxidant and photostable sunscreen functionality in a single molecule to enhance the effectiveness of the antioxidant properties.

SUMMARY OF THE INVENTION

There is provided by the present invention compounds with sunscreen activity, i.e. they are chromophoric within the ultra violet radiation range of from 290-400 nm and they also exhibit antioxidant properties. These compounds are represented by general formula

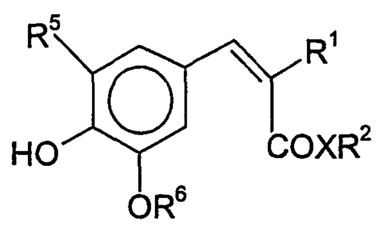

II

For formula II,

$R^1$ is selected from the group consisting of-$C(O)CH_3$, -$CO_2R^3$, -$C(O)NH_2$ and -$C(O)N(R^4)_2$;

X is O or NH;
$R^2$ is linear or branched $C_1$ to $C_{30}$ alkyl;
$R^3$ is linear or branched $C_1$ to $C_{20}$ alkyl;
each $R^4$ is independently hydrogen or linear or branched $C_1$ to $C_8$ alkyl;
$R^5$ is linear or branched $C_1$-$C_8$ alkyl or linear or branched -O-C1-8 alkyl, and
$R^6$ is linear or branched $C_1$-$C_8$ alkyl.

Included within the preferred compounds are those of formula II wherein $R^1$ is linear or branched $C_1$-$C_4$ alkyl, X is oxygen and $R^2$ is linear or branched $C_1$-$C_{12}$ alkyl. Of these compounds, those more preferred have $R^1$ as $C(O)CH_3$ or $CO_2R^3$ wherein $R^3$ is a linear or branched $C_1$ to $C_4$ alkyl. For compounds wherein $R^1$ is $C(O)N(R^4)_2$, $R^4$ is preferably hydrogen or a linear or branched $C_1$-$C_4$ alkyl.

While compounds having from $C_1$-$C_4$ alkyl groups for $R^2$ and $R^3$ are preferred, significant utility can be obtained from compounds wherein $R^2$ and $R^3$ are linear or branched $C_8$ to $C_{20}$ alkyl or $C_{12}$ to $C_{20}$ alkyl groups.

Another preferred class of compounds are those of formulae III and IV wherein $R^1$ and $R^2$ are as defined for formula I with $R^3$ being $C_1$-$C_8$ alkyl and $R^4$ being $C_1$-$C_4$ alkyl.

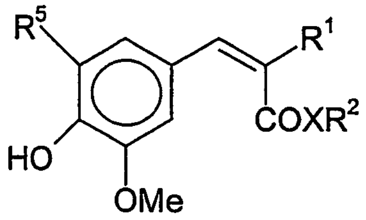

III

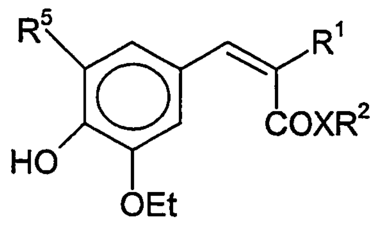

IV

In further preferred compounds -O-$R^6$ and -$R^5$ are identical. Preferably $R^5$ is -O-methyl or -O-ethyl and $R^6$ respectively is -methyl or -ethyl in these compounds.

Another preferred class of compounds are those of formulae V and VI wherein $R^1$ and $R^2$ are as defined for formula I with $R^3$ being $C_1$-$C_8$ alkyl and $R^4$ being $C_1$-$C_4$ alkyl.

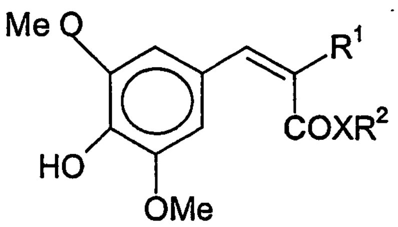

V

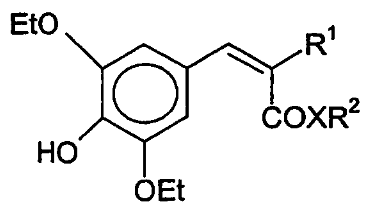

VI

Preferred compounds include those selected from the group consisting of ethyl- alpha- acetyl-3, 5-dimethoxy-4-hydroxy cinnamate, iso-propyl-alpha-acetyl-3,5-dimethoxy-4-hydroxy cinnamate, iso-amyl-alpha-acetyl-3,5-dimethoxy-4-hydroxy cinnamate, 2-ethylhexyl-alpha-acetyl-3,5-dimethoxy-4-hydroxy cinnamate, diethyl-3, 5-dimethoxy- 4-hydroxy benzylidene malonate, di-(2-ethylhexyl)-3,5-dimethoxy- 4-hydroxy benzylidene malonate, diisoamyl-3, 5-dimethoxy-4-hydroxy benzylidene malonate, didodecyl-3,5-dimethoxy-4-hydroxy benzylidene malonate, dipalmitoyl-3, 5-dimethoxy-4-hydroxy benzylidene malonate, di-isopropyl-3,5-dimethoxy-4-hydroxy benzylidene malonate, di-(2-ethyhexyl)-3-methoxy-4-hydroxy-5-isopropyl-benzylidene malonate, di-isoamyl-3-methoxy-4-hydroxy-5-tert.butyl-benzylidene malonate, iso-amyl-alpha-acetyl-3-methoxy-4-hydroxy-5-isopropyl cinnamate, and iso-amyl-alpha-acetyl-3-methoxy-4-hydroxy-5-tert.butyl cinnamate

The present invention also provides sunscreen formulations which comprise a compound of formula II, III and/or IV. These sunscreen formulations are effective in absorbing illumination in the range of wavelengths of 320 nm and above. Amounts of the compounds of formula II, III and/or IV within such compositions typically range from 0.1 to 40

wt% based on the total weight of the sunscreen. These sunscreen formulations can contain one or more additional organic sunscreen agents for filtering UV-B or UV-A rays or they may additionally contain one or more metal oxide sunscreen agents such as titanium dioxide or zinc oxide.

These sunscreen formulations may additionally contain a carrier and at least one component selected from the group consisting of dispersing agents, preservatives, anti-foams, perfumes, oils, waxes, propellants, dyes, pigment emulsifiers, surfactants, thickeners, humectants, exfoliants and emollients. These sunscreen formulations may be in the form of a cosmetic composition with a cosmetically acceptable carrier and one or more cosmetic adjuvants. The sunscreen formulation can optionally have conventional antioxidants or other stabilizers which do not have UV absorbing characteristics.

Methods of using these sunscreen compositions and methods for improving the photostability of sunscreen formulations are also provided. The methods of using the sunscreen formulations comprise applying a sunscreen formulation which contains a compound of formula II, III and/or IV to a substrate. Preferred substrates are skin, hair and fibers. To improve the photostability of a sunscreen formulation, a compound of formula II, III and/or IV is added to the sunscreen formulation in an amount sufficient to reduce the loss of UV absorbance of the sunscreen as it is irradiated. Typical amounts fall within the range of 0.1 % to 40 wt%, based on the total weight of said sunscreen formulation. More typically, the amount falls within the range of 1 wt% to 25 wt%. The amount of organic sunscreen compound of formulae II, III and/or IV, preferably ranges from about 3 wt% to about 15 wt% of the sunscreen formulation. Other ingredients referred to above and discussed more particularly below are generally used in an amount from about 0.1 wt% to about 10 wt% of the sunscreen formulation.The balance comprises a cosmetically or pharmaceutically acceptable carrier.

The sunscreen formulations of this invention preferably offer protection from UV radiation with wavelengths of about 290 nm to 400 nm and preferably from wavelengths in the range of about 290-370 nm. Sunscreen formulations of this invention also typically have a sunscreen protection factor (SPF) ranging from about 2 to 60, with a preferred SPF range of from about 10 to about 45. The target SPF range can be achieved with a combination of both inorganic and organic chromophoric compounds. SPF is determined by techniques well known in the art, on human skin as described in the Federal Register, August 25, 1978, Vol. 43, No. 166, pages 38259-38269 (Sunscreen Drug Products for over-the-counter Human Use, Food and Drug Administration). SPF values can also be approximated using in-vitro models as described, for example, in J. Soc. Cosmet. Chem. 44:127-133 (May/June 1989).

The sunscreen formulations may contain dispersing agents, emulsifiers or thickening agents to assist in applying a uniform layer of the active compounds. Suitable dispersing agents for the sunscreen formulations include those useful for dispersing organic or inorganic sunscreen agents in either a water phase, oil phase, or part of an emulsion, including, for example, chitosan.

Emulsifiers may be used in the sunscreen formulations to disperse one or more of the compounds of formulae I, II, III and/or IV or other component of the sunscreen formulation. Suitable emulsifiers include conventional agents such as, for example, glycerol stearate, stearyl alcohol, cetyl alcohol, dimethicone copolyol phosphate, hexadecyl-D-glucoside, octadecyl-D-glucoside, etc.

Thickening agents may be used to increase the viscosity of the sunscreen formulations. Suitable thickening agents include carbomers, acrylate-/acrylonitrile copolymers, xanthan gum and combinations of these. The carbomer thickeners include the crosslinked CARBOPOL® acrylic polymers from B.F. Goodrich. The amount of thickener within the sunscreen formulation, on a solids basis without water, may range from about 0.001 to about 5%, preferably from 0.01 to about 1% and optimally from about 0.1 to about 0.5% by weight.

Minor optional adjunct ingredients for the sunscreen formulations to be applied to skin or hair may include preservatives, waterproofing agents, fragrances, anti-foam agents, plant extracts (Aloe vera, witch hazel, cucumber, etc) opacifiers, skin conditioning agents and colorants, each in amounts effective to accomplish their respective functions.

The sunscreen formulations may optionally contain an ingredient which enhances the waterproof properties such as, compounds that form a polymeric film, such as dimethicone copolyol phosphate, diisostearoyl trimethyolpropane siloxysilicate, chitosan, dimethicone, polyethylene, polyvinylpyrrolidone (PVP), polyvinylpyrrolidone/vinylacetate,

PVP/Eiconsene copolymer and adipic acids/diethylene glycol/glycerine crosspolymer etc. Waterproofing agents may be present at levels of from about 0.01 to about 10% by weight.

The sunscreen formulations may also optionally contain one or more skin conditioning agents. These include humectants, exfoliants and emollients.

Humectants are polyhydric alcohols intended for moisturizing, reducing scaling and stimulating the removal of built scale from the skin. Typically polyhydric alcohols include polyalkylene glycols and more preferably alkylene polyols and their derivatives. Illustrative are propylene glycol, dipropylene glycol, polypropylene glycol, polyethylene glycol, sorbitol, 2-pyrrolidone-5-carboxylate, hydroxypropyl sorbitol, hexylene glycol, ethoxydiglycol 1,3-butylene glycol, 1,2,6-hexanetriol, glycerin, ethoxylated glycerin, propoxylated glycerin and mixtures thereof. Most preferably the humectant is glycerin. Amounts of humectant can range anywhere from 1 to 30%, preferably from 2 to 20% and optimally from about 5 to 10% by weight of the sunscreen composition.

The exfoliants suitable for use in the present may be selected from alpha-hydroxy carboxylic acids, beta hydroxycarboxylic acids and salts of these acids. Most preferred are glycolic, lactic and salicylic acids and their alkali, metal or ammonium salts.

Suitable emollients include those agents known for softening the skin or hair which may be selected from hydrocarbons, fatty acids, fatty alcohols and esters. Petrolatum is a common hydrocarbon type of emollient conditioning agent. Other hydrocarbons that may be employed include alkyl benzoate, mineral oil, polyolefins such as polydecene, and paraffins, such as isohexadecane. Fatty acids and alcohols typically have from about 10 to 30 carbon atoms. Illustrative are myristic, isostearic, hydroxystearic, oleic, linoleic, ricinoleic, behenic and eruicic acids and alcohols. Oily ester emollients may be those selected from one or more of the following, triglyceride esters, acetoglyceride esters, ethoxylated glycerides, alkyl esters of fatty acids, ether esters, polyhydric alcohol esters and wax esters. Additional emollients or hydrophobic agents include $C_{12}$ to $C_{15}$ alkyl benzoate, dioctyladipate, octyl stearate, octyldodecanol, hexyl laurate, octyldodecyl neopentanoate, cyclomethicone, dicapryl ether, dimethicone, phenyl trimethicone, isopropyl myristate, capriylic/capric glycerides, propylene glycol dicaprylate/dicaprate and decyl oleate.

The sunscreen formulations may optionally contain one or more sunscreen agents as discussed above. In principle, all UV filters are suitable for a combination. Particular preference is given to those UV filters whose physiological safety has already been demonstrated. There are many tried and tested substances known from the specialist literature for both UVA and also UVB filters, e.g.

Benzylidenecamphor derivatives, such as

- 3-(4'-methylbenzylidene)-dl-camphor (e.g. Eusolex® 6300),
- 3-benzylidenecamphor (e.g. Mexoryl® SD),
- polymers of N-{(2 and 4)-[(2-oxoborn-3-ylidene)-methyl]benzyl}acrylamide (e.g. Mexoryl® SW),
- N,N,N-trimethyl-4-(2-oxoborn-3-ylidenemethyl)-anilinium methylsulfate (e.g. Mexoryl® SK) or
- alpha-(2-oxobom-3-ylidene)toluene-4-sulfonic acid (e.g. Mexoryl® SL),

benzoyl- or dibenzoylmethanes, such as

- 1-(4-tert-butylphenyl)-3-(4-methoxyphenyl)propane-1,3-dione (e.g. Eusolex® 9020) or
- 4-isopropyldibenzoylmethane (e.g. Eusolex® 8020),

benzophenones, such as

- 2-hydroxy-4-methoxybenzophenone (e.g. Eusolex® 4360) or
- 2-hydroxy-4.-methoxybenzophenone-5-sulfonic acid and its sodium salt (e.g. Uvinul® MS-40), 4,4,-diarylbutadienes as described in EP-A-0 916 335,

methoxycinnamic esters, such as

- octyl methoxycinnamate (e.g. Eusolex® 2292),
- isopentyl 4-methoxycinnamate, e.g. as a mixture of the isomers (e.g. Neo Heliopan® 1000),

salicylate derivatives, such as

- 2-ethylhexyl salicylate (e.g. Eusolex® OS),
- 4-isopropylbenzyl salicylate (e.g. Megasol®) or
- 3,3,5-trimethylcyclohexyl salicylate (e.g. Eusolex® HMS), 4-aminobenzoic acid and derivatives, such as
- 4-aminobenzoic acid,
- 2-ethylhexyl 4-(dimethylamino)benzoate (e.g. Eusolex® 6007),
- ethoxylated ethyl 4-aminobenzoate (e.g. Uvinul® P25),

diphenylacrylates, e.g. 2-ethylhexyl-2-cyano-3,3-diphenylacrylate (Eusolex® OCR)
and further substances, such as 2-phenylbenzimidazole-5-sulfonic acid, and its potassium, sodium and triethanolamine salts (e.g. Eusolex® 232), 3,3'-(1,4-phenylenedimethylene)-bis(7,7-dimethyl-2-oxobicyclo[2.2.1]hept-1-ylmethanesulfonic acid and its salts (e.g. Mexoryl® SX), 2,4,6-trianilino-(p-carbo-2'-ethylhexyl-1'-oxy)-1,3,5-triazine (e.g. Uvinul® T 150) and 2-(4-Diethylamino-2-hydroxy-benzoyl)-benzoic acid hexyl ester (e.g. Uvinul□ A Plus, BASF).

The compounds given in the list are only to be regarded as examples. It is of course also possible to use other UV filters.

These organic UV filters are usually incorporated into cosmetic formulations in an amount of from 0.5 to 10% by weight, preferably 1 - 8%.

Further suitable organic UV filters are, for example, 2-(2H-benzotriazol-2-yl)-4-methyl-6-(2-methyl-3-(1,3,3,3-tetramethyl-1-(trimethylsilyloxy)-disiloxanyl)propyl)phenol (e.g. Silatrizole®), bis(2-ethylhexyl) 4,4'-[(6-[4-((1,1-dimethylethyl)—aminocarbonyl)phenylamino]-1,3,5-triazine-2,4-diyl)-diimino]-bis-benzoate (e.g. Uvasorb® HEB), (trimethylsilyl)-[trimethylsilyl)oxy]-poly-[oxy-(dimethyl [and about 6% methyl[2-[p-[2,2-bis-(ethoxycarbonyl]vinyl]phenoxy]-1-methylenethyl] and about 1.5% methyl[3-[p-[2,2-bis(ethoxycar-bonyl)-vinyl)phenoxy)propenyl) and 0.1 to 0.4% (methyl-hydrogen]silylene]] (n□60) (CAS No. 207 574-74-1), 2,2'-methylenebis(6-(2H-benzotriazol-2-yl)-4-(1,1,3,3-tetramethylbutyl) phenol) (CAS No. 103 597-45-1), 2,2'-(1,4-phenylene)bis(1H-benzimidazol-4,6-disulfonic acid, monosodium salt) (CAS No. 180 898-37-7) and 2,4-bis{[4-(2-ethylhexyloxy)-2-hydroxyl]phenyl}-6-(4-methoxyphenyl)-1,3,5-triazine (CAS No. 103 597-45-, 187 393-00-6).

These organic UV filters are usually incorporated into cosmetic formulations in an amount of from 0.5 to 20% by weight, preferably 1 - 15%.

Conceivable as inorganic UV filters are those from the group of titanium dioxides, such as, for example, coated titanium dioxide (e.g. Eusolex® T-2000, Eusolex® T-AQUA), zinc oxides (e.g. Sachtotec®), iron oxides and also cerium oxides. These inorganic UV filters are generally incorporated into cosmetic formulations in an amount of from 0.5 to 20% by weight, preferably 2 - 10%.

Preferred compounds with UV-filtering properties are 3-(4'-methylbenzylidene)-dl-camphor, 1-(4-tert-butyl-phenyl)-3-(4-methoxyphenyl)-propane-1,3-dione, 4-iso-propyldi-benzoylmethane, 2-hydroxy-4-methoxy-benzo-phenone, octyl methoxy-cinnamate, 3,3,5-trimethylcyclohexyl salicylate, 2-ethylhexyl 4-(dimethylamino)-benzoate, 2-ethylhexyl 2-cyano-3,3-diphenylacrylate, 2-phenyl-benzimidazole-5-sulfonic acid and its potassium, sodium and triethanolamine salts.

By combining one or more compounds of the formula I with further UV filters it is possible to optimize the protective action against harmful effects of UV radiation.

All of the UV filters specified herein including the compounds of formula I can also be used in encapsulated form. In particular, it is advantageous to use organic UV filters in encapsulated form. Specifically, the following advantages arise:

- The hydrophilicity of the capsule wall can be set independently of the solubility of the UV filter. First, for example, it is possible to incorporate even hydrophobic UV filters into purely aqueous formulations. In addition, the oily impression which is often perceived as unpleasant upon application of the preparation comprising hydrophobic UV filters is prevented.
- Certain UV filters, in particular dibenzoylmethane derivatives, exhibit only reduced photostability in cosmetic formulations. By encapsulating these filters or compounds which impair the photostability of these filters, such as, for example, the above-mentioned cinnamic acid derivatives, it is possible to increase the photostability of the overall formulation.
- The literature discusses again and again the penetration of the skin by organic UV filters and the irritancy potential associated therewith upon direct application to the human skin. The encapsulation of the corresponding substances proposed here presents this effect.
- Generally, by encapsulating individual UV filters or other ingredients, it is possible to avoid formulation problems which arise as a result of interaction of individual formulation constituents with one another, such as crystallization processes, precipitations and agglomeration, since the interaction is prevented.

It is therefore preferred according to the invention if one or more of the above-mentioned UV filters are present in encapsulated form. In this connection, it is advantageous if the capsules are so small that they can not be observed with the naked eye. To achieve the above-mentioned effects, it is also necessary for the capsules to be sufficiently stable and not to release the encapsulated active ingredient (UV filter) into the surroundings, or to release it only to a slight extent.

Suitable capsules can have walls made of inorganic or organic polymers. For example, US 6,242,099 B1 describes the preparation of suitable capsules with balls made of chitin, chitin derivatives or polyhydroxylated polyamines. Capsules which are to be used particularly preferably according to the invention have walls which can be obtained by a sol-gel process, as is described in the applications WO 00/09652, WO 00/72806 and WO 00/71084. Preference is given here in turn to capsules whose walls are made of silica gel (silica; undefined silicon oxide hydroxide). The preparation of corresponding capsules is known to the person skilled in the art, for example, from the cited patent applications, the contents of which also expressly belonging to the subject-matter of the present application.

Here, the capsules are present in formulations according to the invention preferably in amounts which ensure that the encapsulated UV filters are present in the formulation in the amounts given above.

The protecting action against oxidative stress or against the effect of free radicals can be further improved if the formulation comprises one or more antioxidants.

There are many tried and tested substances known from the specialist literature which can be used, e.g. amino acids (e.g. glycine, histidine, tyrosine, tryptophan) and derivatives thereof, imidazoles (e.g. urocanic acid) and derivatives

thereof, peptides, such as D,L-camosine, D-camosine, L-camosine and derivatives thereof (e.g. anserine), carotinoids, carotenes (e.g. □-carotene, □-carotene, lycopene) and derivatives thereof, chlorogenic acid and derivatives thereof, lipoic acid and derivatives thereof (e.g. dihydrolipoic acid), aurothioglucose, propylthiouracil and other thiols (e.g. thioredoxin, glutathione, cysteine, cystine, cystamine and the glycosyl, N-acetyl, methyl, ethyl, propyl, amyl, butyl and lauryl, palmitoyl, oleyl, □-linoleyl, cholesteryl and glycerylesters thereof), and salts thereof, dilauryl thiodipropionate, distearyl thiodipropionate, thiodipropionic acid and derivatives thereof (esters, ethers, peptides, lipids, nucleotides, nucleosides and salts), and sulfoximine compounds (e.g. buthioninesulfoximine, homocysteine-sulfoximine, buthionine-sulfone, penta-, hexa-and heptathionine-sulfoximine) in very low tolerated doses (e.g. pmol to μmol/kg), and also (metal) chelating agents, (e.g. α-hydroxy fatty acids, palmitic acid, phytic acid, lactoferrin), α-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof, vitamin C and derivatives (e.g. ascorbyl palmitate, magnesium ascorbyl phosphate, ascorbyl acetate), tocopherols and derivatives (e.g. vitamin E acetate), vitamin A and derivatives (e.g. vitamin A palmitate), and coniferyl benzoate of benzoin resin, rutin and salts of the sulfuric ester of rutin and derivatives thereof, α-glycosyl rutin, ferulic acid, furfurylidineglucitol, carosine, butylhydroxy-toluene, butylhydroxyanisol, nordihydroguaretic acid, trihydroxybutyrophenone, quercetin, uric acid and derivatives thereof, mannose and derivatives thereof, zinc and derivatives thereof (e.g. ZnO, ZnSO4), selenium and derivatives thereof (e.g. selenomethionine), stilbenes and derivatives thereof (e.g. stilbene oxide, trans-stilbene oxide).

Mixtures of antioxidants are likewise suitable for use in the cosmetic formulations according to the invention. Known and commercial mixtures are, for example, mixtures comprising, as active ingredients, lecithin, L-(+)-ascorbyl palmitate and citric acid (e.g. Oxynex® AP), natural tocopherols, L-(+)-ascorbyl palmitate, L-(+)-ascorbic acid and citric acid (e.g. Oxynex® K LIQUID), tocopherol extracts from natural sources, L-(+)-ascorbyl palmitate, L-(+)-ascorbic acid and citric acid (e.g. Oxynex® L LIQUID), DL-□-tocopherol, L-(+)-ascorbyl palmitate, citric acid and lecithin (e.g. Oxynex® LM) or butylhydroxytoluene (BHT), L-(+)-ascorbyl palmitate and citric acid (e.g. Oxynex® 2004).

The formulations according to the invention can comprise vitamins as further ingredients. Preferably, vitamins and vitamin derivatives chosen from vitamin A, vitamin A propionate, vitamin A palmitate, vitamin A acetate, retinol, vitamin B, thiamine chloride hydrochloride (vitamin B1), riboflavin (vitamin B2) nicotinamide, vitamin C (ascorbic acid), vitamin D, ergocalciferol (vitamin D2), vitamin E, DL-tocopherol, tocopherol E acetate, tocopherol hydrogen-succinate, vitamin K1, esculin (vitamin P active ingredient), thiamine (vitamin B1) nicotinic acid (niacin), pyridoxine, pyridoxal, pyridoaxmine, (vitamin B6), panthothenic acid, biotin, folic acid and cobalamine (vitamin B12) are present in the cosmetic formulations according to the invention, particularly preferably vitamin A palmitate, vitamin C, DL-tocopherol, tocopherol E acetate, nicotinic acid, panthothenic acid and biotin.

The composition of our invention can be a cosmetic formulation or a pharmaceutical formulation.

Examples of application forms of the cosmetic or pharmaceutical formulations according to the invention which may be mentioned are: solutions, suspensions, emulsions, PIT emulsions, pastes, ointments, gels, creams, lotions; powders, soaps, foams, surfactant-containing cleansing preparations, oils, aerosols and sprays. Examples of other application forms are sticks, shampoos and shower preparations. Any customary carriers, auxiliaries and optionally further active ingredients may be added to the formulation.

Preferred auxiliaries originate from the group of preservatives, antioxidants, stabilizers, solubility promoters, vitamins, colorants, odour improvers.

Ointments, pastes, creams and gels may comprise the customary carriers, e.g. animal and vegetable fats, waxes, paraffins, starch, tragacanth, cellulose derivatives, polyethylene glycols, silicones, bentonites, silica, talc and zinc oxide or mixtures of these substances.

Powders and sprays may comprise the customary carriers, e.g. lactose, talc, silica, aluminium hydroxide, calcium silicate and polyamide powder or mixtures of these substances. Sprays can additionally comprise customary propellants, e.g. chlorofluorocarbons, propane/butane or dimethyl ether.

Solutions and emulsions can comprise the customary carriers, such as solvents, solubility promoters and emulsifiers, e.g. water, ethanol, isopropanol, ethyl carbonate, ethyl acetate, benzyl alcohol, benzyl benzoate, propylene glycol, 1,3-butylglycol, oils, in particular cotton seed oil, peanut oil, wheatgerm oil, olive oil, castor oil and sesame oil, glycerol fatty acid ester, polyethylene glycols and fatty acid esters of sorbitan or mixtures of these substances.

Suspensions can comprise the customary carriers such as liquid diluents, e.g. water, ethanol or propylene glycol, suspending agents, e.g. ethoxylated isostearyl alcohols, polyoxyethylene sorbitol esters and polyoxyethylene sorbitan esters, microcrystalline cellulose, aluminium metahydroxide, bentonite, agar agar and tragacanth or mixtures of these substances.

Soaps can comprise the customary carriers, such as alkali metal salts of fatty acids, salts of fatty acid mono esters, fatty acid protein hydrolysates, isethionates, lanolin, fatty alcohol, vegetable oils, plant extracts, glycerol, sugars or mixtures of these substances.

Surfactant-containing cleansing products can comprise the customary carrier substances, such as salts of fatty alcohol sulfates, fatty alcohol ether sulfates, sulfosuccinic monoesters, fatty acid protein hydrolysates, isethionates,

imidazolinium derivatives, methyl taurates, sarcosinates, fatty acid amide ether sulfates, alkylamidobetaines, fatty alcohols, fatty acid glycerides, fatty acid diethanolamides, vegetable and synthetic oils, lanolin derivatives, ethoxylated glycerol fatty acid esters or mixtures of these substances.

Face and body oils can comprise the customary carrier substances such as synthetic oils, such as fatty acid esters, fatty alcohols, silicone oils, natural oils, such as vegetable oils and oily plant extracts, paraffin oils, lanolin oils or mixtures of these substances.

Further typically cosmetic application forms are also lipsticks, lipcare sticks, mascara, eyeliner, eyeshadow, blusher, powder make-up, emulsion make-up and wax make-up, and sunscreen, presun and aftersun preparations.

All compounds or components which can be used in the cosmetic formulations are either known and available commercially or can be synthesized by known processes.

As dispersant or solubilizer it is possible to use an oil, wax or other fatty substance, a lower monoalcohol or a lower polyol or mixtures thereof. Preferred monoalcohols or polyols include ethanol, isopropanol, propylene glycol, glycerol and sorbitol.

A preferred embodiment of the invention is an emulsion in the form of a protective cream or milk and which comprise, for example, fatty alcohols, fatty acids, fatty acid esters, in particular triglycerides of fatty acids, lanolin, natural and synthetic oils or waxes and emulsifiers in the presence of water.

Further preferred embodiments are oily lotions based on natural or synthetic oils and waxes, lanolin, fatty acid esters, in particular triglycerides of fatty acids, or oily-alcoholic lotions based on a lower alcohol, such as ethanol, or a glycerol, such as propylene glycol, and/or a polyol, such as glycerol, and oils, waxes and fatty acid esters, such as triglycerides of fatty acids.

The cosmetic preparation according to the invention can also be in the form of an alcoholic gel which comprises one or more lower alcohols or polyols, such as ethanol, propylene glycol or glycerol, and a thickener, such as siliceous earth. The oily-alcoholic gels also comprise natural or synthetic oil or wax.

Preferred compositions of our invention are hydrogels. The hydrophilicity of the capsule wall can be set independently of the solubility of the UV filter. For example, it is possible to incorporate even hydrophobic UV filters into purely aqueous formulations. Due to this possibility to include high amounts of hydrophobic UV filters in encapsulated or immobilized form, as described above, those hydrogels of our inventions can posses high SPF values, in a range normally only acheived with oily formulations.

The solid sticks consist of natural or synthetic waxes and oils, fatty alcohols, fatty acids, fatty acid esters, lanolin and other fatty substances.

All compounds or components which can be used in the cosmetic or pharmaceutical formulations are either known and available commercially or can be synthesized by known processes.

The composition according to the invention is particularly suitable for protecting human skin against the harmful influences of the UV constituents in sunlight, in addition they also offer protection against ageing processes of the skin and against oxidative stress, i.e. against damage caused by free radicals, as are produced, for example, by solar irradiation, heat or other influences.

Therefore the use of a composition according to our invention for the manufacture of a medicament suitable for the prophylaxis of damages of the skin caused by sunray, especially for the prophylaxis of sunburn and sun-caused erythrema is another embodiment of our invention. A further embodiment is the cosmetic prophylaxis of damages of the skin caused by sunray, especially for the prophylaxis of sunburn and sun-caused erythrema.

The formulation may comprise adjuvants which are customarily used in this type of composition, such as, for example, thickeners, softeners, moisturizers, surface-active agents, emulsifiers, preservatives, perfumes, waxes, lanolin, propellants, dyes and/or pigments which colour the composition itself or the skin, and other ingredients customarily used in cosmetics.

The composition may be a foamable composition able to be foamed up with or without an propellant. According to our invention it is especially preferred if the foam is produced without the use of a organic propellant. Sprays using organic propellents may not be stored in direct sun or at higher temperatures; conditions that for example can often be found on the beach during summer. An advantage of preferred compositions according to our invention is that the may be stored and used even under these conditions.

Preferred compositions are included in a foam dispenser, preferably in a foam dispenser that requires no organic propellant as described above.

As foam builders / stabilisers in general all substances able to build or stabilise a foam may be used. Those substances in general are known to those skilled in the art. Preferred foam builders / stabilisers are those which are skin tolerant or even more preferably give a benefit to the skin.

The foam builders / stabilisers are usually present in an amount of about 0.01 to 20 % by weight, preferably in an amount of 0.1 to 5 % by weight and even more preferred in an amount of 0.1 to 3 % by weight.

Preferred foam builders / stabilisers are cetyl phosphate, DEA cetyl phosphate, TEA myristate, TEA stearate, magnesium stearate, sodium stearate, potassium laurate, potassium ricinoleate, sodium cocoate, sodium tallowate,

potassium castorate, sodium oleate and mixtures thereof.

Other preferred foam stabilisers are so called foam boosters. Foam boosters are substances which increase the surface viscosity of the liquid which surrounds the individual bubbles in a foam. These agents are commonly used in shaving soaps, shampoos, bubble baths, liquid soaps, mousses, or aerosol-dispensed foams. Also Film Formers or Viscosity-Increasing Agents maybe used as foam boosters. The listing below gives examples for foam boosters which can also be classified as surfactants (INCI names):

Acetamide MEA, Almondamide DEA, Almondamidopropylamine Oxide, Almondamidopropyl Betaine, Apricotamide DEA, Apricotamidopropyl Betaine, Avocadamide DEA, Avocadamidopropyl Betaine, Babassuamide DEA, Babassuamidopropylamine Oxide, Babassuamidopropyl Betaine, Behenamide DEA, Behenamide MEA, Behenamidopropyl Betaine, Behenamine Oxide, Behenyl Betaine, Canolamidopropyl Betaine, Capramide DEA, Camitine, Cetearyl Alcohol, Cetyl Alcohol, Cetyl Betaine, Cocamide DEA, Cocamide MEA, Cocamide MIPA, Cocamidoethyl Betaine, Cocamidopropylamine Oxide, Cocamidopropyl Betaine, Cocamidopropyl Hydroxysultaine, Cocamine Oxide, Cocoamphodipropionic Acid, Cocobetainamido Amphopropionate, Coco-Betaine, Coco-Hydroxysultaine, Coco-Morpholine Oxide, Coconut Alcohol, Coco/Oleamidopropyl Betaine, Coco-Sultaine, Cocoyl Sarcosinamide DEA, DEA-Cocoamphodipropionate, DEA-Lauraminopropionate, Decyl Alcohol, Decylamine Oxide, Decyl Betaine, Diethanolaminooleamide DEA, Dihydroxyethyl C8-10 Alkoxypropylamine Oxide, Dihydroxyethyl C9-11 Alkoxypropylamine Oxide, Dihydroxyethyl C12-15 Alkoxypropylaminde Oxide, Dihydroxyethyl Cocamine Oxide, Dihydroxyethyl Lauramine Oxide, Dihydroxyethyl Stearamine Oxide, Dihydroxyethyl Tallowamine Oxide, Dimethicone Propyl PG-Betaine, Disodium Caproamphodiacetate, Disodium Caproamphodipropiante, Disodium Capryloamphodiacetate, Disodium Capryloamphodipropionate, Disodium Cetearyl SulfosuccinateDisodium Cocamido MIPA-Sulfosuccinate, Disodium Cocamido PEG-3 Sulfosuccinate, Disodium Cocaminopropyl Iminodiacetate, DisodiumCocoamphocarboxyethylhydroxypropylsulfonate, Disodium Cocoamphodiacetate, Disodium Cocoamphodipropionate, Disodium C12-15 Pareth Sulfosuccinate, Disodium Deceth-5 Sulfosuccinate, Disodium Deceth-6 Sulfosuccinate, Disodium Hydrogenated Cottonseed Glyceride Sulfosuccinate, Disodium Isodecyl Sulfosuccinate, Disodium Isostearamido MEA-Sulfosuccinate, Disodium Isostearamido MIPA-Sulfosuccinate, Disodium Isostearoamphodiacetate, Disodium Isostearoamphodipropionate, Disodium Isostearyl Sulfosuccinate, Disodium Laneth-5 Sulfosuccinate, Disodium Lauramido MEA-Sulfosuccinate, Disodium Lauramido PEG-2 Sulfosuccinate, Disodium Laureth-5 Carboxyamphodiacetate, Disodium Laureth Sulfosuccinate, Disodium Laureth-6 Sulfosuccinate, Disodium Laureth-9 Sulfosuccinate, Disodium Laureth-12 Sulfosuccinate, Disodium Lauroamphodiacetate, Disodium Lauroamphodipropiante, Disodium Lauryl Sulfosuccinate, Disodium Myristamido MEA-Sulfosuccinate, Disodium Nonoxynol-10 Sulfosuccinate, Disodium Oleamido MEA-Sulfosuccinate, Disodium Oleamido MIPA-Sulfosuccinate, Disodium Oleamido PEG-2 Sulfosuccinate, Disodium Oleoamphodipropionate, Disodium Oleth-3 Sulfosuccinate, Disodium Oleyl Sulfosuccinate, Disodium Palmitamido PEG-2 Sulfosuccinate, Disodium Palmitoleamido PEG-2 Sulfosuccinate, Disodium PEG-4 Cocamido MIPA-Sulfosuccinate, Disodium PPG-2-Isodeceth-7 Carboxyamphodiacetate, Disodium Ricinoleamido MEA-Sulfosuccinate, Disodium Stearamido MEA-Sulfosuccinate, Disodium Stearoamphodiacetate, Disodium Stearyl Sulfosuccinamate, Disodium Stearyl Sulfosuccinate, Disodium Tallamido MEA-Sulfosuccinate, Disodium Tallowamido MEA-Sulfosuccinate, Disodium Tallowamphodiacetate, Disodium Tallow Sulfosuccinamate, Disodium Tridecylsulfosuccinate, Disodium Undecylenamido MEA-Sulfosuccinate, Disodium Undecylenamido PEG-2 Sulfosuccinate, Disodium Wheat Germamido MEA-Sulfosuccinate, Disodium Wheat Germamido PEG-2 Sulfosuccinate, Disodium Wheatgermamphodiacetate, Di-TEA-Oleamido PEG-2 Sulfosuccinate, Ditridecyl Sodium Sulfosuccinate, Erucamidopropyl Hydroxysultaine, Hydrogenated Tallow Alcohol, Hydrogenated Tallowamide DEA, Hydrogenated Tallowamine Oxide, Hydrogenated Tallow Betaine, Hydroxyethyl Carboxymethyl Cocamidopropylamine, Hydroxyethly Hydroxypropyl C12-15 Alkoxypropylamine Oxide, Hydroxystearamide MEA, Isostearamide DEA, Isostearamide MEA, Isostearamide MIPA, Isostearamidopropylamine Oxide, Isostearamidopropyl Betaine, Isostearamidopropyl Morpholine Oxide, Lactamide MEA, Lanolinamide DEA, Lauramide DEA, Lauramide MEA, Lauramide MIPA, Lauramide/Myristamide DEA, Lauramidopropylamine Oxide, Lauramidopropyl Betaine, Lauramine Oxide, Lauroampho-dipropionic Acid, Lauryl Alcohol, Lauryl Betaine, Lauryl Hydroxysultaine, Lauryl Sultaine, Lecithinamide DEA, Linoleamide DEA, Linoleamide MEA, Linoleamide MIPA, Methyl Morpholine Oxide, Minkamide DEA, Minkamidopropylamine Oxide, Minkamidopropyl Betaine, Myristamide DEA, Myristamide MEA, Myristamide MIPA, Myristamidopropylamine Oxide, Myristamidopropyl Betaine, Myristamine Oxide, Myristaminopropionic Acid, Myristyl Alcohol, Myristyl Betaine, Myristyl/Cetyl Amine Oxide, Oleamide DEA, Oleamide MEA, Oleamide MIPA, Oleamidopropylamine Oxide, Oleamidopropyl Betaine, Oleamidopropyl Hydroxysultaine, Oleamine Oxide, Oleyl Betaine, Olivamide DEA, Olivamidopropylamine Oxide, Olivamidopropyl Betaine, Palmamide DEA, Palmamide MEA, Palmamide MIPA, Palmamidopropyl Betaine, Palmitamide DEA, Palmitamide MEA, Palmitamidopropylamine Oxide, Palmitamidopropyl Betaine, Palmitamine Oxide, Palm Kernel Alcohol, Palm Kernelamide DEA, Palm Kernelamide MEA, Palm Kernelamide MIPA, Palm Kemelamidopropyl Betaine, Peanutamide MEA, Peanutamide MIPA, PEG-3 Cocamide, PEG-2 Hydrogenated Tallow Amine, PEG-3 Lauramide, PEG-3 Lauramide Oxide, PEG-2 Oleamide, PEG-3 Oleamide, PEG-2 Oleamine, PEG-2 Soyamine, PEG-2 Stearamine, Potassium Dihydroxyethyl Cocamine Oxide Phosphate, Ricinoleamide DEA, Ricinoleamide MEA, Ricinoleamide MIPA, Ricinoleamidopropyl Betaine, Sesamide DEA, Sesamidopropylamine Oxide, Sesamidopropyl Betaine, Sodium Caproamphoacetate, Sodium

Caproamphohydroxy-propylsulfonate, Sodium Caproamphopropionate, Sodium Capryloampho-acetate, Sodium Capryloamphohydroxypropylsulfonate, Sodium Capryloamphoproprionate, Sodium Cocoamphoacetate, Sodium Cocoamphohydroxypropylsulfonate, Sodium Cocoamphopropionate, Sodium Comamphopropionate, Sodium Isostearoamphoacetate, Sodium Isostearoamphopropionate, Sodium Lauramidopropyl Hydroxyphostaine, Sodium Lauraminopropionate, Sodium Lauriminodipropionate, Sodium Lauroamphoacetate, Sodium/MEA Laureth-2 Sulfosuccinate, Sodium Myristoamphoacetate, Sodium Oleoamphoacetate, Sodium Oleoampho-hydroxy-propylsulfonate, Sodium Oleoamphopropionate, Sodium Ricinoleoamphoacetate, Sodium Stearoamphoacetate, Sodium Stearoamphohydroxypropylsulfonate, Sodium Stearoamphopropionate, Sodium Tallamphopropionate, Sodium Tallowamphoacetate, Sodium Tallowate, Sodium Undecylenoampho-acetate, Sodium Undecylenoampho-propionate, Sodium Wheat Germamphoacetate, Soyamide DEA, Soyamidopropyl Betaine, Stearamide AMP, Stearamide DEA, Stearamide DEA-Distearate, Stearamide MEA, Stearamide MEA-Stearate, Stearamide MIPA, Stearamidopropylamine Oxide, Stearamidopropyl Betaine, Stearamine Oxide, Stearyl Alcohol, Stearyl Betaine, Tallamide DEA, Tallowamide DEA, Tallowamide MEA, Tallowamidopropylamine Oxide, Tallowamidopropyl Betaine, Tallowamidopropyl Hydroxysultaine, Tallowamine Oxide, Tallow Betaine, TEA-Lauraminopropionate, TEA-Myristaminopropionate, Trideceth-2 Carboxamide MEA, Trisodium Lauroampho PG-Acetate Phosphate Chloride, Undecylenamide DEA, Undecylenamide MEA, Undecylenamidopropylamine Oxide, Undecylenamidopropyl Betaine, Wheat Germamide DEA, Wheat Germamidopropylamine Oxide, Wheat Germamidopropyl Betaine.

In another embodiment at least one foam builder / stabiliser is selected from foaming surfactants, preferably from alkylglyosides, anionic protein derivatives or fatty acid sulfonates.

An especially preferred foam builder / stabilisers are anionic protein derivatives, such as lipoaminoacids described in WO 98/09611, WO 99/27902 and WO 99/45899. Most preferred under these anionic protein derivatives are sodium lauroyl oat amino acids, for example known as Proteol™ Oat (Tradename of Seppic).

The cosmetic formulation can also be used to protect the hair against photochemical damage in order to prevent changes of colour shades, decoloration or damage of a mechanical nature. In this case, a suitable formulation is in the form of a shampoo or lotion for rinsing out, the formulation in question being applied before or after shampooing, before or after colouring or bleaching or before or after permanent waving. It is also possible to choose a formulation in the form of a lotion or gel for styling or treating the hair, in the form of a lotion or gel for brushing or blow-waving, in the form of a hair lacquer, permanent waving composition, colorant or bleach for the hair. The cosmetic formulation may comprise various adjuvants used in this type of composition, such as surface-active agents, thickeners, polymers, softeners, preservatives, foam stabilizers, electrolytes, organic solvents, silicone derivatives, antigrease agents, dyes and/or pigments which colour the composition itself or the hair, or other ingredients customarily used for hair care.

To protect the skin and/or natural or sensitized hair against solar rays, the cosmetic composition is applied to the skin or the hair. Sensitized hair is understood here as meaning hair which has been subjected to a chemical treatment, such as a permanent waving treatment, a colouring process or bleaching process.

Sunscreen formulations of this invention as described in Formulations 8-16 can be prepared by conventional means.

Formulation 8: Oil/Water Sunscreen Lotion

| INCI Name | Trade Name/Manufacturer | % w/w |
|---|---|---|
| **Phase A** | | |
| Avobenzone | Eusolex 9020/Rona | 3.00 |
| Disoamyl-3-methoxy-4-hydroxy-5-isopropyl benzylidene malonate | | 3.00 |
| Isopropyl Myristate | Emerest 2314/Henkel | 4.00 |
| C12-15 Alkyl Benzoate | Finsolv TN/Finetex | 4.00 |
| Cetyl Alcohol | Crodacol C-70/Croda | 1.50 |
| Steareth- 2 | Bnj 72/ICI Surfactants | 2.00 |
| Steareth- 21 | Bnj 721/ICI Surfactants | 2.50 |
| Dimethicone | Dow Corning Fluid 200, 100sct/Dow | 0.50 |
| **Phase B** | | |
| Deionized Water | | 78.07 |
| Acrylayes/C10-30 Alkyl Acrylates Crosspolymer | Carbopol ETD 2020/BF Goodrich | 0.20 |

(continued)

| | | |
|---|---|---|
| **Phase C** | | |
| Triethanolamine (99%) | TEA 99% /Union Carbide | 0.23 |
| **Phase D** | | |
| Phenoxyethanol (and) isopropylparaben (and)isobutylparaben (and) butylparaben | Liquapar PE/Sutton | 1.00 |
| **Total** | | **100.00** |

Procedure

Preapare Phase B by dispersing Carbopol in water. Heat the dispersion to 70-75°C.
Combine Phase A ingredients. Stir and heat to 70-75°C.
Add Phase B to Phase A while stirring.
Add Phase C. Homogenize until mixture cools to 45-40°C.
Add Phase D. Stir allowing mixture to cool to room temperature.

Formulation 9: Oil/Water Sunscreen Lotion

| **INCI Name** | **Trade Name/Manufacturer** | **% w/w** |
|---|---|---|
| **Phase A** | | |
| Avobenzone | Eusolex 9020/Rona | 3.00 |
| Iso-amyl-alpha-acetyl-3-methoxy-4-hydroxy 5-isopropyl cinnamate | | 3.00 |
| Isopropyl Myristate | Emerest 2314/Henkel | 4.00 |
| C12-15 Alkyl Benzoate | Finsolv TN/Finetex | 4.00 |
| Cetyl Alcohol | Crodacol C-70/Croda | 1.50 |
| Steareth- 2 | Bnj 72/ICI Surfactants | 2.00 |
| Steareth-21 | Bnj 721/ICI Surfactants | 2.50 |
| Dimethicone | Dow Coming Fluid 200, 100sct/Dow | 0.50 |
| **Phase B** | | |
| Deionized Water | | 78.07 |
| Acrylayes/C10-30 Alkyl Acrylates Crosspolymer | Carbopol ETD 2020/BF Goodrich | 0.20 |
| **Phase C** | | |
| Triethanolamine (99%) | TEA 99% /Union Carbide | 0.23 |
| **Phase D** | | |
| Phenoxyethanol (and) isopropylparaben (and)isobutylparaben (and) butylparaben | Liquapar PE/Sutton | 1.00 |
| **Total** | | **100.00** |

Procedure

Preapare Phase B by dispersing Carbopol in water. Heat the dispersion to 70-75°C.
Combine Phase A ingredients. Stir and heat to 70-75°C.
Add Phase B to Phase A while stirring.
Add Phase C. Homogenize until mixture cools to 45-40°C.
Add Phase D. Stir allowing mixture to cool to room temperature.

FORMULATION 10

| Phase A | | Phase B | |
|---|---|---|---|
| Deionized water | 60.0 % | Ethyl-alpha-acetyl-3,5-dimethoxy-4- hydroxy cinnamate | 8.75% |
| Disodium EDTA | 0.10 % | Octyl salicylate | 5 % |
| Glycerin | 1.5 % | Aluminum stearate | 5 % |
| NaCl | 3.0 % | Cyclomethicone/dimethicone | 10 % |
| Butylene glycol | 2.5 % | Cetyl dimethicone | 1 % |
| | | Cyclomethicone | 2 % |
| | | ABIC- EM 97 | 1 % |
| | | Fragrance | .15 % |

Procedure

Combine phase B ingredients. Stir and heat to 70-75°C. Combine Phase A ingredients. Heat while stirring to 70-75°C. Add Phase B to Phase A while stirring. Add preservative. Stir, allowing mixture to cool to room temperature.

Formulation 11: Sunscreen Oil/Water Spray Lotion

| **INCI Name** | **Trade Name (Supplier)** | **% w/w** |
|---|---|---|
| **Phase A-1** | | |
| Di-isopropyl-3,5-dimethoxy-4- hydroxybenzylidene malonate | | 7.50 |
| Benzophenone-3 | Eusolex® 4360 (Rona) | 2.50 |
| Dicapryl ether | Cetiol® OE (Henkel) | 4.50 |
| Dimethicone | Dow Coming 200®, 50 cst (Dow) | 2.00 |
| Stearyl Alcohol | Crodacol S-70 (Croda) | 0.60 |
| PPG-2 Ceteareth-9 | Eumulgin® L (Henkel) | 0.40 |
| Steareth-10 | Volpo 10 (Croda) | 0.50 |
| Glyceryl stearate, PEG-100 Stearate | Arlacel® 165 (ICI) | 2.80 |
| **Phase A-2** | | |
| Titanium Dioxide, Simethicone, Alumina | Eusolex® T-2000 (Rona) | 5.00 |
| **Phase B-1** | | |
| Demineralized water | | 66.10 |
| Chitosan, water | Hydagen® CMF (Henkel) | 2.00 |
| Glycerin USP | Emery 916 (Henkel) | 2.50 |
| Dimethicone copolyol phosphate | Pecosil PS-100 (Phoenix Chemical) | 2.50 |
| **Phase B-2** | | |
| Polyquatemium 37, Mineral oil, PPG-1 trideceth-6 | Salcare SC 95 (Ciba) | 0.40 |

(continued)

| Phase C | | |
|---|---|---|
| Propylene Glycol, DMDM Hydantoin, Methylparaben, Propylparaben | Paragon™ II (McIntyre) | 0.70 |
| **Total** | | **100.00** |

Procedure

Combine A-1; stir and heat to 60 °C until allsolids are dissolved. Disperse A-2 in A-1 with agitation. Combine B-1; stir and heat to 60 °C. Disperse B-2 in B-1 with agitation. Add A to B while stirring vigorously. gently homogenize allowing mixture to cool to 40 °C. Add C to A/B: gently homogenize until mixture is uniform. Stir with anchor mixer allowing to reach 25 °C prior to packaging. Use a high shear pump spray device for dispensing (e.g., Eurogel pump by Seaquist Perfect)

Formulation 12: Sunscreen Cream

| INCI Name | Trade Name/Manufacturer | % w/w |
|---|---|---|
| **Phase A** | | |
| Deionized water | | 39.73 |
| Carbomer (2% aq. solution) | Carbopol 980/BF Goodrich | 15.00 |
| Propylene Glycol | | 5.00 |
| Methylparaben | | 0.20 |
| Propylparaben | | 0.10 |
| Triethanolamine (99%) | | 0.45 |
| Tetrasodium EDTA | | 0.02 |
| **Phase B** | | |
| Octyl Methoxycinnamate | Eusolex® 2292/Rona | 5.00 |
| Benzophenone-3 | Eusolex® 44360/Rona | 3.00 |
| Di-isoamyl-3,5-dimethoxy-4- hydroxybenzylidene malonate | | 4.50 |
| Glyceryl Stearate (and) PEG-100 Stearate | Ariacel 165/ICI Surfactants | 1.00 |
| Cyclomethicone | Dow Corning 344 Fluid/Dow Coming | 5.00 |
| Glyceryl Stearate | | 4.00 |
| Stearic Acid | Emersol 132, NF/Henkel | 2.50 |
| Isostearyl Isostearate | Prisonne ISIS 2039/Unichema | 10.00 |
| Hydrogenated Castor Oil | Castorwax/CasChem | 2.00 |
| $C_{12-15}$ Alcohols Benzoate | Finsolv TN/Finetex | 2.50 |
| **Total** | | **100.00** |

Procedure

Add Phase A ingredients to main vessel under impeller agitation. Heat phase A to 75-80°C. Combine Phase B ingredients; heat and mix to 85°C. Slowly add Phase B to batch; mix for 15 minutes at 85°C. Remove from heat; switch to paddle mixing and cool to room temperature.

Formulation 13: Water/Oil Broad Spectrum Sunscreen Lotion

| INCI Name | Trade Name/Manufacturer | % w/w |
|---|---|---|
| **Phase A-1** | | |
| Octyl Methoxycinnamate | Eusolex® 2292/Rona | 7.50 |
| Iso-amyl-alpha-acetyl-3,5-dimethoxy-4- hydroxy-cinnamate | | 5.00 |
| Octyl Stearate | Cetiol 868/Henkel | 2.00 |
| Dicapryl Ether | Cetiol OE/Henkel | 3.00 |
| Cyclomethicone | Dow Coming 345 Fluid/Dow Coming | 4.00 |
| Dimethicone | DC 200 fluid 50cST/Dow Coming | 2.00 |
| PEG-30 Dipolyhydroxystearate | Ariacel P135/ICI | 1.30 |
| Laurylmethicone copolyol | Dow Coming formulation Aid 5200/Dow | 2.30 |
| Behenamidopropyl dimethylamine Behenate | Catemol 220-B/Phoenix Chemical | 0.50 |
| **Phase A-2** | | |
| Titanium Dioxide (and) Alumina (and) Simethicone | Eusolex® T-2000/Rona | 8.00 |
| Deionized Water | | 61.00 qs |
| Propylene Glycol | | 2.00 |
| Sodium Chloride | | 0.80 |
| **Phase C** | | |
| DMDM Hydantoin, Methylparaben, Propylparaben | Paragon II/Mclntyre | 0.60 |
| **Total** | | **100.00** |

Procedure

Combine A-1; stir and heat to 55-60°C until all solids are dissolved. Disperse A-1 in A-1 by propeller agitation. Combine B; stir and heat to 50-55°C. Slowly add B to A while stirring vigorously. Add C to A/B; gently homogenize until mixture is uniform. Stir with anchor mixer allowing mixture to cool to room temperature.

Formulation 14: UVA/UVB Sun Protection Cream with Avobenzone

| INCI Name | Trade Name/Manufacturer | % w/w |
|---|---|---|
| **Phase A-1** | | |
| Water (demineralized) | | 67.80 |
| Disodium EDTA | | 0.05 |
| Propylene glycol | | 3.00 |
| Methylparaben | | 0.15 |
| **Phase A-2** | | |
| Carbomer | Carbopol Ultrez 10/BF Goodrich | 0.20 |
| **Phase B** | | |
| Isopropyl Myristate | | 2.00 |
| Cetyl Alcohol, Glyceryl Stearate, PEG-75 Stearate, Ceteth 20, Steareth 20 | Emulium Delta/Gattefosse | 4.00 |

(continued)

| | | |
|---|---|---|
| **Phase B** | | |
| Diethyl-3,5-dimethoxy-4-hydroxybenzylidene malonate | | 3.50 |
| Homomethyl salicylate | Eusolex® HMS/Rona | 7.00 |
| Octyl salicylate | Eusolex® OS/Rona | 7.00 |
| Butyl methoxydibenzoylmethane | Eusolex® 9020/Rona | 3.00 |
| Dimethicone | Dow Coming Fluid 200, 100sct/Dow | 1.00 |
| C30-38 Olefin/isopropyl Maleate/MA Copolymer | Performa V 1608/New Phase Technologies | 1.00 |
| **Phase C** | | |
| Triethanolamine (99%) | | 0.30 |
| **Phase D** | | |
| preservatives | | q.s. |
| **Total** | | **100.00** |

Procedure

Combine A-1; heat to 50°C while stirring until methylparaben is dissolved. Disperse A-2 in A-1 with a sifter. Heat A to 65°C
Combine B; heat to 65-70°C while stirring until solids are dissolved.
Add B to A. Homogenize Add C at 55-60°C. Continue to homogenize allowing mixture to cool to 40-45°C.
Add D; stir with propeller mixer until uniform.
Adjust pH with TEA to 6.5-7.0.

Formulation 15: Oil/Water Sunscreen Lotion

| **INCI Name** | **Trade Name/Manufacturer** | **% w/w** |
|---|---|---|
| **Phase A** | | |
| Diisoamyl-3,5-dimethoxy-4- hydroxybenzylidene malonate | | 3.00 |
| Isopropyl Myristate | Emerest 2314/Henkel | 4.00 |
| C12-15 Alkyl Benzoate | Finsolv TN/Finetex | 4.00 |
| Cetyl Alcohol | Crodacol C-70/Croda | 1.50 |
| Steareth- 2 | Bnj 72/ICI Surfactants | 2.00 |
| Steareth- 21 | Bnj 721/ICI Surfactants | 2.50 |
| Dimethicone | Dow Coming Fluid 200, 100sct/Dow | 0.50 |
| **Phase B** | | |
| Deionized Water | | 81.07 |
| Acrylates/C10-30 Alkyl Acrylates Crosspolymer | Carbopol ETD 2020/BF Goodrich | 0.20 |
| **Phase C** | | |
| Triethanolamine (99%) | TEA 99% /Union Carbide | 0.23 |

(continued)

| | | |
|---|---|---|
| **Phase D** | | |
| Phenoxyethanol (and) isopropylparaben (and) isobutylparaben (and) butylparaben | Liquapar PE/Sutton | 1.00 |
| **Total** | | **100.00** |

Procedure

Prepare Phase B by dispersing Carbopol in water. Heat the dispersion to 70-75°C.
Combine Phase A ingredients. Stir and heat to 70-75°C.
Add Phase B to Phase A while stirring.
Add Phase C. Homogenize until mixture cools to 45-40°C.
Add Phase D. Stir allowing mixture to cool to room temperature.

Formulation 16: Oil/Water Sunscreen Lotion

| INCI Name | Trade Name/Manufacturer | % w/w |
|---|---|---|
| **Phase A** | | |
| Avobenzone | Eusolex 9020/Rona | 3.00 |
| Diisoamyl-3,5-dimethoxy-4-hydroxy-benzylidene malonate | | 3.00 |
| Isopropyl Myristate | Emerest 2314/Henkel | 4.00 |
| C12-15 Alkyl Benzoate | Finsolv TN/Finetex | 4.00 |
| Cetyl Alcohol | Crodacol C-70/Croda | 1.50 |
| Steareth- 2 | Bnj 72/ICI Surfactants | 2.00 |
| Steareth- 21 | Bnj 721/ICI Suttactants | 2.50 |
| Dimethicone | Dow Coming Fluid 200, 100sct/Dow | 0.50 |
| **Phase B** | | |
| Deionized Water | | 78.07 |
| Acrylayes/C10-30 Alkyl Acrylates Crosspolymer | Carbopol ETD 2020/BF Goodrich | 0.20 |
| **Phase C** | | |
| Triethanolamine (99%) | TEA 99% /Union Carbide | 0.23 |
| **Phase D** | | |
| Phenoxyethanol (and) isopropylparaben (and)isobutylparaben (and) butylparaben | Liquapar PE/Sutton | 1.00 |
| **Total** | | **100.00** |

Procedure

Preapare Phase B by dispersing Carbopol in water. Heat the dispersion to 70-75°C.
Combine Phase A ingredients. Stir and heat to 70-75°C.
Add Phase B to Phase A while stirring.
Add Phase C. Homogenize until mixture cools to 45-40°C.
Add Phase D. Stir allowing mixture to cool to room temperature.

It has been found that to provide antioxidant functionality, the phenyl group of the compounds of formula I should have a substituent patterns of "3,5-alkoxy-4-hydroxy or 3-alkoxy-4-hydroxy-5-alkyl."

The compounds of Formula II-IV, V and VI can be obtained by condensation of a corresponding 3, 5-dialkoxy, 4-hydroxy benzaldehyde of formula B,

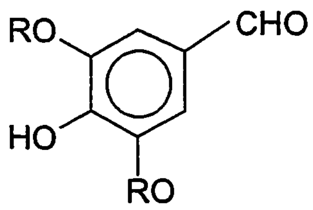

B

wherein R is as defined above, with a compound that provides the UV absorbing moiety as defined above. An example is a compound of the formula: $R^1$-$CH_2C(O)XR^2$
wherein $R^1$ and $R^2$ and X are as defined above for formulae II-IV.

The benzaldehyde of formula B can be obtained commercially or prepared from 3, 4, 5-trimethoxybenzaldehyde through selective monodemethylation at the 4-position. This technique leads to syringaldehyde. Alternately, syringaldehyde can be prepared from 5-bromo-vanillin by replacing bromo-group with methoxy ( or alkoxy or alkyl) using appropriate alcohol or activated alkyl functionality. The syringaldehyde or other aldehydes is then condensed with a compound to provide the desired UV absorbing moiety.

## EXAMPLES

Example XI: Disoamyl-3-methoxy-4-hydroxy-5-isopropyl benzylidene malonate

Condensation of 3-methoxy-4-hydroxy-5-isopropyl benzaldehyde with di-isoamyl malonate in the presence of piperidine-acetic acid and benzene as the reaction medium at reflux temperature under continuous azeotropic water removal yields the title product. The reaction takes about 3 hours for completion. The yield obtained is typically 90-95%.

Example XII: Isoamyl-alpha-acetyl-3-methoxy-4-hydroxy-5-isopropylcinnamate

Condensation of 3-methoxy-4-hydroxy-5-isopropyl benzaldehyde with isoamyl acetoacetate in the presence of piperidine-acetic acid and benzene as the reaction medium at reflux temperature under continuous azeotropic water removal yields the title product. The reaction takes about 4 hrs for completion. The yield obtained is typically 90-95%.

Example XIV: Diethyl-3,5-dimethoxy-4-hydroxy benzylidene malonate

Monodemethylation of 3,4,5-trimethoxy benzaldehyde using sulphuric acid at 40°C. for 8 hours yields syringaldehyde.

Condensation of 3,5-Dimethoxy-4-hydroxy benzaldehyde (Syringaldehyde) with diethyl malonate in the presence of piperidine - acetic acid and benzene as the reaction medium at reflux temperature under continuous azeotropic water removal yields the title product. The reaction takes about 7.5 hours for completion.

Example XV: Ethyl-alpha-methyl-3,5-dimethoxy-4-hydroxy cinnamate

Monodemethylation of 3,4,5,-trimethoxy benzaldehyde using sulphuric acid at 40°C for 8 hours yields syringaldehyde.
The Wittig salt is prepared by reaction of triphenyl phosphine and ethyl-2-bromopropionate in benzene media at 70-75°C for 8 hours and subsequent basification with 1 N Sodium hydroxide to phenolphthalein end point at room temperature. Extraction with benzene, concentration of the benzene extract and the addition of petroleum ether (60-80°C) yield triphenyl methyl carbethoxy methylene phosphorane as a solid product. Condensation of 3,5-Dimethoxy-4-hydroxy benzaldehyde (Syringaldehyde) with triphenyl methyl carbethoxy methylene phosphorane is performed at reflux temperature in xylene for seven hours and after work up, yields the title compound.

Example XVI: Ethyl-alpha-acetyl-3,5-dimethoxy-4-hydroxy-cinnamate

Monodemethylation of 3,4,5-trimethoxy benzaldehyde using sulphuric acid at 40°C for 8 hours yields syringaldehyde.
Condensation of 3,5-Dimethoxy-4-hydroxy benzaldehyde (syringaldehyde) with ethyl acetoacetate in the presence of piperidine - acetic acid and benzene as the reaction medium at reflux temperature yields the title product. The reaction

takes about 3.5 hours for completion.

Example XVII: Di-(2-Ethylhexyl)-3,5-dimethoxy-4-hydroxy benzylidene malonate

Monodemethylation of 3,4,5-trimethoxy benzaldehyde using sulphuric acid at 40°C for 8 hours yields syringaldehyde.
Transesterfication of diethyl malonate using 2-ethylhexyl alcohol in neat condition at 140-155°C for 2 hours under nitrogen blanketing in the presence of sulphuric acid and after work up, followed by high vacuum distillation, yields di-6-ethylhexyl malonate.
Condensation of 3,5-Dimethoxy-4-hydroxy benzaldehyde (Syringaldehyde) with di-2-ethylhexyl malonate in the presence of piperidine - acetic acid and benzene as the reaction medium at reflux temperature under continuous azeotropic water removal yields di-2-ethylhexyl-3,5-dimethoxy-4-hydroxy benzylidene malonate. The reaction takes about nine hours for completion. The yield typically obtained is 91 %.

Example XVIII: Di-isoamyl-3,5-dimethoxy-4-hydroxy benzylidene malonate

Example XIV was repeated, except in the condensation step, diethyl malonate was replaced with di-isoamyl malonate. The yield typically obtained was over 90%.

Example XIX: Di-isopropyl-3,5-dimethoxy-4-hydroxy benzylidene malonate

Example XIV was repeated, except in the condensation step, diethyl malonate was replaced with di-isopropyl malonate. The yield typically obtained was over 90%.

Example XX: Di-dodecyl-3,5-dimethoxy-4-hydroxy benzylidene malonate

Example XIV was repeated, except in the condensation step, diethyl malonate was replaced with di-dodecyl malonate. The yield typically obtained was over 90%.

Example XXI: Iso-propyl-alpha-acetyl-3,5-dimethoxy-4-hydroxy-cinnamate

Example XVI was repeated, except in the condensation step, ethyl acetoacetate was replaced with iso-propyl acetoacetate. The yield of the desired product was 88%.

Example XXII: Iso-butyl-alpha-acetyl-3,5-dimethoxy-4-hydroxy-cinnamate

Example XVI was repeated, except in the condensation step, ethyl aceto-acetate was replaced with iso-butyl-acetoacetate. The yield of the desired product was 89%.

Example XXIII: Iso-amyl-alpha-acetyl-3,5-dimethoxy-4-hydroxy-cinnamate

Example XVI was repeated, except in the condensation step, ethyl acetoacetate was replaced with iso-amyl acetoacetate. The yield of the desired product was 89%.

Comparative Example A : Ethyl-alpha-cyano-3,4,5-trimethoxy cinnamate

Condensation of 3,4,5-trimethoxy benzaldehyde with ethyl cyanoacetate in the presence of piperidine - acetic acid and benzene as the reaction medium at reflux temperature under continuous azeotropic water removal yields the title product. The reaction takes about three hours for completion and the yield obtained is typically 90%.

Comparative Example B: Diethyl-3,4,5-trimethoxy benzylidene malonate

Condensation of 3,4,5-trimethoxy benzaldehyde with diethyl malonate in the presence of piperidine - acetic acid and benzene as the reaction medium at reflux temperature under continuous azeotropic water removal yields the title product. The reaction takes about ten hours for completion. The yield obtained is typically 85%

Antioxidant Activity by DPPH Test Method

A DPPH concentrate (2.5X) of 25mg of 1,1-Diphenyl-2-Picyrl-Hydrazyl ACS# 1898-66-4 (Sigma #D-9132, lot 99H3601) dissolved in 250mL ethanol (USP), is prepared fresh on the measurement date. A DPPH working solution is then prepared by diluting 100mL of this concentrate to a final volume of 250mL (100 ΦM/mL). A blank 13x100mm borosilicate glass screw top tube of ethanol (USP) is used to zero the spectrometer (Milton Roy, Spectronic 20+) at 517 nm and a control tube of DPPH working solution is measured under identical conditions, and taken as 0% activity. Aliquots of the 0.25% & 0.5% (RT & 45EC) test solution are added to tubes followed by the rapid addition of 4mL DPPH working solution then rapidly capped and mixed. After 20 minutes, the absorbance of each sample is read at 517 nm.

The percent reducing activity (%RA) is calculated using the following equation:

$$\% \text{ Reduction Activity} = \frac{100\ A(0)-A(20)}{A(0)}$$

Where A(0) is the absorbance value of the DPPH working solution at 517nm zeroed against an ethanol blank and A(20) is the absorbance at 517nm, 20 minutes after combining the antioxidant with the DPPH working solution.

The concentration of antioxidant (mg/ml) in the final assay mixture is calculated based on the dilution of respective aliquots of each compound in the final assay volume and %RA tabulated and plotted as percent activity at each concentration in the dilution series.

Antioxidant Property

The antioxidant activity of selected compounds of this invention was determined from their reducing activity of a DPPH radical. Results of selected compounds from this invention are summarized in the Tables 1 and 2.

Table 1: Antioxidant Activity of Vanillin-based Compounds of this Invention

| Compounds | %Reducing Activity of DPPH Radical at 30 μg/ml | μg/ml Needed to Reduce 50% of DPPH radical ($IC_{50}$) |
|---|---|---|
| Di-(2-ethylhexyl)-3-methoxy-4-hydroxy benzylidene malonate * | 7.4 | 188 |
| Di-isoamyl-3-methoxy-4-hydroxy benzylidene malonate * | 9.5 | 172 |
| Isoamyl-alpha-acetyl-3-methoxy-4-hydroxy cinnamate * | 22 | 72 |
| Di-(2-ethylhexyl)-3-methoxy-4-hydroxy-5-isopropyl-benzylidene malonate | 11.2 | 161 |
| Isoamyl-alpha-acetyl-3-methoxy-4-hydroxy-5-isopropyl cinnamate | 28 | 68 |
| Tinogard | | 46 |
| * not according to the invention | | |

Table 2 shows the antioxidant property of Syringaldehyde-based compounds (2/1, 2/2, 2/3, 2/4, 2/5, 2/7, 2/8 and 2/10).

| mglml | 2/1 | 2/2 | 2/3 | 2/4 | 2/5 | 2/7 | 2/8 | 2/10 |
|---|---|---|---|---|---|---|---|---|
| 2.500 | 00 | 3 | 25.40 | 71.70 | | | | |
| 0.278 | | | 15.5 | 33.4 | 59.9 | 85.0 | 33.7 | 30.1 |
| 0.139 | | | 11.3 | 29.51 | 47.3 | 77.3 | 23.2 | 22.3 |
| 0.056 | | | 8.4 | 12.8 | 31.0 | 61.2 | 13.0 | 14.6 |

(continued)

| mglml | 2/1 | 2/2 | 2/3 | 2/4 | 2/5 | 2/7 | 2/8 | 2/10 |
|---|---|---|---|---|---|---|---|---|
| 0.028 | | | 5.2 | 5.2 | 20.7 | 46.1 | 8.5 | 10.2 |

2/1 diethyl-3,4,5-trimethoxy benzylidene malonate*
2/2 ethyl-alpha-cyano-3,4,5-trimethoxy cinnamate*
2/3 ethyl-alpha-cyano-3,5-dimethoxy-4-hydroxy cinnamate*
2/4 diethyl-3,5-dimethoxy-4-hydroxy benzylidene malonate
2/5 ethyl-alpha-acetyl-3,5-dimethoxy-4-hydroxy cinnamate
2/7 ethyl-alpha-methyl-3,5-dimethoxy-4-hydroxy cinnamate
2/8 di(2-ethyl-hexyl)-3,5-dimethoxy-4-hydroxy benzylidene malonate
2/10 di-iso-amyl-3,5-dimethoxy-4-hydroxy benzylidene malonate
* not according to the invention

Compounds with 3,5-dimethoxy-4-hydroxy substitution were found to exhibit much higher reducing activity (antioxidant activity) than compounds with 3,4,5-trimethoxy substitution. In order to boost antioxidant activity of the compounds of the present invention, other antioxidants can be combined. Some examples are those antioxidants mentioned above and Tocopherols, tocopherylacetate, Ascorbic acid, Emblica antioxidants, Proanthocyanidins (from pine bark, grape seed extract, and the like) green tea polyphenols, rosemary antioxidants, gallic acid, ellagic acid, butylhydroxy toluene (BHT), butylhydroxy anisole (BHA) and the like.

Photostability

The photostability of selected compounds (see Table 3 and 4) were tested according to the procedures below.

A solar simulator used for illumination of the samples in the experiments is constructed incorporating a 1 kw Xe arc lamp, optical bench and sample illumination chamber. The lamp output is filtered through a water filter with a course window to remove most of the infrared radiation and optical filters to remove wavelengths below 290 nm. The output of the illumination system is focused onto the face of a 1 cm quartz Cuvette that is thermally equilibrated with a constant temperature water bath at 25°C. A magnetic stir is mounted under the Cuvette so that the samples could be stirred while being illuminated. An electric shutter is controlled by a dark room timer to provide precise control of illumination times. The solar simulator is constructed to provide illumination that closely matches terrestrial sunlight. The solar simulator delivers roughly 250 $J/cm^2$ over a 2-hour period of illumination in a 290-490 nm range. This irradiance is determined using two nitrobenzaldehyde chemical actinometry. The irradiance is much higher than other solar simulator systems which typically illuminate a large area in order to illuminate many samples simultaneously rather than being focused down to a very small area.

Each sunscreen compound is dissolved in 70% ethanol/30% water and the UV visible absorption spectrum measured with a Shimadza UV 2101-double beam spectrophotometer using the solvent as reference. A control solution of Octocrylene is prepared and the UV-visible absorption spectrum measured. Each solution is then illuminated for two hours in the solar simulator. After illumination, the absorption spectrum is again measured for each solution.

As Table 3 and 4 illustrate, the tested compounds were found to be photostable after two hours of illumination in a Xe-arc solar simulator. This data shows compounds of this invention have comparable photostabilities to Octocrylene under the experimental conditions employed.

Photostability of the individual compounds is determined by differential UV-absorption spectra before and after illumination. % Loss of absorption, hence the loss of individual compound, is calculated from the reduction in optical density after illumination. Likewise, stabilization of Avobenzone in the presence of individual compounds of this invention was also calculated.

Table 3: Results of Photostability of Vanillin-Based Compounds of this Invention & their Stabilization to Avobenzone (in solution)

| Compound | $\lambda_{max}$, nm (Ethanol) | Photostability % Loss in 2 hrs | Stabilization of Avobenzone[2] % Loss of Avobenzone in 2 hrs |
|---|---|---|---|
| Di-(2-ethylhexyl)-3-methoxy-4-hydroxy benzylidene malonate* | 332 | 1 | 4.5 |

(continued)

| Compound | $\lambda_{max}$, nm (Ethanol) | Photostability % Loss in 2 hrs | Stabilization of Avobenzone[2] % Loss of Avobenzone in 2 hrs |
|---|---|---|---|
| Di-isoamyl-3-methoxy-4-hydroxy benzylidene malonate * | 333 | 3.2 | 3.8 |
| Isoamyl-alpha-acetyl-3-methoxy-4-hydroxy cinnamate * | 339 | 4.1 | Insignificant |
| Di-(2-ethylhexyl)-3-methoxy-4-hydroxy-5-isopropyl-benzylidene malonate | 334 | 2 | 2.5 |
| Isoamyl-alpha-acetyl-3-methoxy-4-hydroxy-5-isopropyl cinnamate | 341 | 3.2 | Insignificant |
| Avobenzone | 358 | 37 | |
| Octocrylene (control) | 303 | 2.8 | 1.5 |

* not according to the invention
[1]Solvent used 70% ethanol/30% water; solar simulator; about 250 $J/cm^2$
[2]Product/Avobenzone (1:1, w/w); %Loss of Avobenzone by HPLC

All tested syringaldehyde-based compounds were found to be photostable after two hours of illumination in a Xe-arc solar simulator with the exception of two compounds 2/7. An examination of the UV-visible spectra (Table 4) reveals that the 2/1, 2/4, 2/5, 2/8, 2/10, 2/20, 2/22, 2/24 and 2/25 sunscreen compounds retain most of their absorptivity after 2 hours of illumination in the solar simulator while 2/7 degraded significantly (72% loss of absorbance) during this illumination period. The Octocrylene solution was found to exhibit a very little loss in absorptivity after 2 hours of illumination in the solar simulator. Thus 2/1, 2/4, 2/5, 2/8, 2/10, 2/20, 2/22, 2/24 and 2/25 have comparable photostabilities to Octocrylene under the experimental conditions employed.

Table 4: Results of Photostability of Syringaldehyde-Based Compounds of this Invention & their Stabilization to Avobenzone (in solution)

| Compounds | Photostability Loss in 2 hrs[1] | Loss of Avobenzone in 2 hrs[2] |
|---|---|---|
| Diethyl-3,4,5-trimethoxy benzylidene malonate (2/1) | 0% | No loss |
| Diethyl-3,5-dimethoxy-4-hydroxy benzylidene malonate (2/4) | 3% | No loss |
| Ethyl-alpha-methyl-3,5-dimethoxy-4- hydroxy cinnamate (2/7) | 72% | Not done |
| Di-isopropyl-3,5-dimethoxy-4-hydroxy benzylidene malonate (2/20) | 1% | Not done |
| Di-isoamyl-3,5-dimethoxy-4-hydroxy benzylidene malonate (2/10) | 4.1 % | 6% |
| Di-(2-ethylhexyl)-3,5-dimethoxy-4-hydroxy benzylidene malonate (2/8) | 4.1%[6] | 2% |
| Di-dodecyl-3,5-dimethoxy-4-hydroxy benzylidene malonate (2/24) | 2% | 19% |

(continued)

| Compounds | Photostability Loss in 2 hrs[1] | Loss of Avobenzone in 2 hrs[2] |
|---|---|---|
| Ethyl-alpha-acetyl-3,5-dimethoxy-4- hydroxycinnamate (2/5) | 1% | 10% |
| Isoamyl-alpha-acetyl-3,5-dimethoxy-4- hydroxycinnamate (2/22) | 4% | 4% |
| Isobutyl-alpha-acetyl-3,5-dimethoxy-4- hydroxycinnamate (2/25) | 5% | Not done |
| Avobenzone | 37% | |
| Di-2-ethylhexyl-2,6-napthalene dicarboxylic acid (DENDA) | 4% | 6% |
| Octocrylene | 5% | 1.5% |

[1] Solvent used 70% ethanol / 30% water, solar simulator, about 250 $J/cm^2$, % product loss
[2] Product / Avobenzone (1:1, w/w); % Loss of Avobenzone by HPLC

Stabilizing Activity

The stabilizing activity of selected compounds (2/1, 2/4, 2/5, 2/8, 2/10, 2/22, 2/24) toward Avobenzone was tested and compared with a conventional product according to the procedures below.

Individual solutions of selected sunscreen compounds (2/1, 2/4, 2/5, 2/8, 2/10, 2/22, 2/24) with Avobenzone were as follows. Each sunscreen compound was dissolved in 70% ethanol/30% $H_2O$ solution containing roughly an equal molar amount of Avobenzone. A similar solution containing Di-2-ethylhexyl-2,6-napthalene dicarboxylic acid (DENDA) or octocrylene and Avobenzone were also prepared. Each solution was then illuminated in the solar simulator as configured above for the photostability tests and aliquots of each solution were removed at 30-minute time intervals. These aliquots were injected into an HPLC and the loss of Avobenzone was followed with illumination time. The high performance liquid chromatograph (HPLC) used for all experiments reported therein incorporated a Spectra-Physics model P-200 pump with an Applied Biosystems model 785A UV-Visible detector with a Rheodyne manual injector incorporating a 50 ml sample loop and a 150 x 4.6 mm reversed-phase $C_{18}$ column (Alltech). All analyses were carried out under isocratic elution conditions using $CH_3OH/H_2O$ mixtures for the mobile phase at a flow rate of 1 $H_2O$ ml per minute. It was necessary to employ HPLC separation of Avobenzone from each of the sunscreen compounds to quantify Avobenzone due to the absorption spectra overlap with some of these compounds. Results are shown in the Table 4.

The loss of Avobenzone when illuminated alone in solution was rapid exhibiting a half life of approximately 3 hours in the solar simulator. Figs. 3 and 4 reveal that the sunscreen compounds appear to stabilize Avobenzone as effectively as DENDA antioxidant. The loss of Avobenzone in the presence of DENDA was found to follow unusual kinetics. Initially the loss of Avobenzone was quite rapid but then seemed to become stable.

The UV-spectral study of the present inventive compounds shows that they have broad absorption bands that extend across the UV region. They exhibit lower molar absorption than Avobenzone but have much better photostability than Avobenzone. General data regarding the absorbance characteristics of the sunscreen compounds and Avobenzone are presented in Table 5.

Table 5: Absorbance and molar absorptivity data for sunscreen compounds measured in 70% ethanol/30% $H_2O$ solution

| Compound | $\lambda_{Max}$ | $\lambda_{Max}(M^{-1}cm^{-1})$ |
|---|---|---|
| Avobenzone | 360 nm | $2.5 \times 10^4$ |
| Diethyl,3,4,5-trimethoxy benzylidene malonate (2/1) | 313 nm | $2.0 \times 10^4$ |
| Ethyl-alpha-cyano 3,4,5- trimethoxy cinnamte (2/2) | 338 nm | $1.2 \times 10^4$ |
| Ethyl-alpha-acetyl 3,5,- dimethoxy-4-hydroxy cinnamte (2/5) | 348 nm | $1.9 \times 10^4$ |
| Ethyl-alpha-methyl 3,5- dimethoxy-4-hydroxy cinnamte (2/7) | 314 nm | N/A |

(continued)

| Compound | $\lambda_{Max}$ | $\lambda_{Max}(M^{-1}cm^{-1})$ |
|---|---|---|
| Di-(2-ethylhexyl)-3,5- dimethoxy-4-hydroxy benzylidene malonate (2/8) | 340 nm | $1.6 \times 10^4$ |

Photostabilization of avobenzone in formulations under UV B light

Surprisingly, we have found that photostabilzation of Avobenzone with compounds of present invention is much superior to commercially available photostabilzer (DENDA or TQ). Thus three formulations have been tested for Avobenzone photostability : Avobenzone (2%), Avobenzone + 2/8 (2%:2%, [di-(2-ethylhexyl)3,5-dimethoxy-4-hydroxy benzylidene malonate]) and Avobenzone + TQ (2%:2%). Tests were performed by obtaining a thick product films between 2 glass slides and then irradiated for 8 h (about 16 MED, 8mJ/$Cm^2$) under UV B light. A small amount of product is then re-dissolved in Ethanol:water (70%:30%) and the maximum absorbance measured. % residual Avebenzone was calculated by differential UV spectra. Result is summarized in the Table 6.

Table 6: Stabilization of avobenzone in formulated products

| Formulated Products | Intitial Avobenzone | Avobenzone left after 8 hr of UV-B irradiation |
|---|---|---|
| Avobenzone | 100% | 27% |
| Avobenzone+ 2/8 | 100% | 81% |
| Avobenzone + TQ | 100% | 21% |

Photostabilization of Avobenzone (2%) in formulation under UV B irradiation at various ratios of photostabilizer (2-6%): 2/8, TQ and OCR

For better visualization of the long-term photostabilization effects of 2/8 [di-(2-ethylhexyl)3,5-dimethoxy-4-hydroxy benzylidene malonate], samples have been tested by measuring the transmission spectra. From these spectra, absorption at 358 nm was corrected for light scattering and overlapping absorption bands. The relative loss of avobenzone is calculated from the relative decrease in this absorption band. Results are summarized in the Table 7.

Table 7: Stabilization of avobenzone in formulated products with different ratios of stabilizers

| Formulated Products | Intitial Avobenzone | Avobenzone left after 8 hr of UV-B irradiation |
|---|---|---|
| Avobenzone | 100% | 27% |
| Avobenzone+ 2/8 (2%/2%) | 100% | 81% |
| Avobenzone+ 2/8 (2%/4%) | 100% | 84% |
| Avobenzone+ 2/8 (2%/6%) | 100% | 94% |
| Avobenzone+ TQ (2%/2%) | 100% | 21% |
| Avobenzone+ TQ (2%/4%) | 100% | 27% |
| Avobenzone + TQ (2%/6%) | 100% | 26% |

The preceding examples can be repeated with similar success by substituting the generically or specifically described reactants and/or operating conditions of this invention for those used in the examples.

## Claims

**1.** A compound of formula II

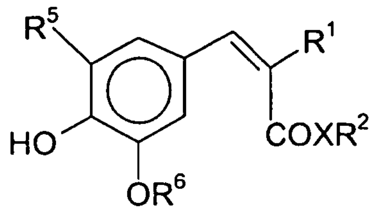

II

wherein

$R^1$ is selected from the group consisting of $-C(O)CH_3$, $-CO_2R^3$, $- C(O)NH_2$ and $-C(O)N(R^4)_2$;
X is O or NH;
$R^2$ is linear or branched $C_1$ to $C_{30}$ alkyl;
$R^3$ is linear or branched $C_1$ to $C_{20}$ alkyl; and
each $R^4$ is independently hydrogen, or linear or branched $C_1$ to $C_8$ alkyl;
$R^5$ is linear or branched $C_1$-$C_8$ alkyl or linear or branched $-O-C_{1-8}$ alkyl,
and $R^6$ is $C_1$ to $C_8$ alkyl.

**2.** A compound of claim 1 wherein $R^6$ is $C_1$-$C_8$ alkyl, X is oxygen and $R^2$ is linear or branched $C_1$ to $C_4$ alkyl.

**3.** A compound of at least one of the claims 1 or 2 wherein $R^1$ is $CO_2R^3$ and, $R^3$ is linear or branched $C_1$ to $C_8$ alkyl, preferably $C_1$ to $C_4$ alkyl.

**4.** A compound of at least one of the claims 1 to 3 wherein $R^1$ is $C(O)CH_3$.

**5.** A compound of at least one of the claims 1 or 2 wherein $R^1$ is $-C(O)N(R^4)_2$, and at least one $R^4$ is hydrogen and the other is hydrogen or linear or branched $C_1$ to $C_4$ alkyl.

**6.** A compound of at least one of the claims 1 or 2 wherein $R^1$ is $-C(O)N(R^4)_2$, and each $R^4$ is independently linear or branched $C_1$ to $C_4$ alkyl.

**7.** A compound of claim 1 wherein $R^6$ is $C_1$-$C_4$ alkyl, $R^1$ is $-CO_2R^3$, and at least one of $R^2$ and $R^3$ is linear or branched $C_8$ to $C_{20}$ alkyl.

**8.** A compound of claim 7 wherein $R^2$ and $R^3$ are each linear or branched $C_8$-$C_{12}$ alkyl.

**9.** A compound of claim 7 wherein at least one of $R^2$ and $R^3$ is linear or branched $C_{12}$ to $C_{20}$ alkyl.

**10.** A compound of at least one of the claims 1 to 9 wherein $R^6$ is methyl or ethyl.

**11.** A compound according to claim 1, wherein the compound is one of the formulae III or IV

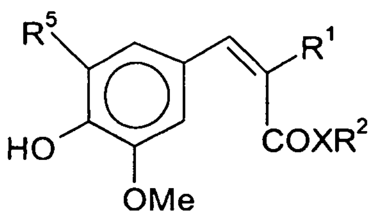

III

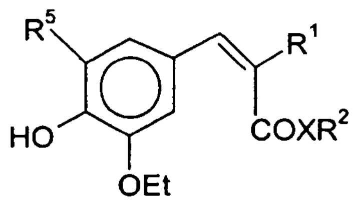

IV

wherein

$R^1$ is selected from the group consisting $-C(O)CH_3$, $-CO_2$ ($C_1$-$C_8$ alkyl), $-C(O)NH_2$ and $-C(O)N(C_1$-$C_4$ alkyl$)_2$;
X is O or NH; and
$R^2$ is $C_1$-$C_{12}$ alkyl, and
$R^5$ is $C_1$-$C_8$ linear or branched alkyl or -O- $C_{1-8}$ linear or branched alkyl.

**12.** A compound of claim 11 wherein X is oxygen and $R^2$ is linear or branched $C_1$ to $C_4$ alkyl and $R^1$ is $-CO_2C_8H_{18}$.

**13.** A compound of at least one of the claims 1 to 12, wherein -O-$R^6$ and -$R^5$ are identical, and preferably $R^5$ is -O-Methyl or -O-Ethyl and $R^6$ is -Methyl or -Ethyl.

**14.** A compound of claim 1 selected from the group consisting of di-(2-ethyhexyl)-3-methoxy-4-hydroxy-5-isopropyl-benzylidene malonate, di-isoamyl-3-methoxy-4-hydroxy-5-tert.butyl-benzylidene malonate, iso-amyl-alpha-acetyl-3-methoxy-4-hydroxy-5-isopropyl cinnamate, iso-amyl-alpha-acetyl-3-methoxy-4-hydroxy-5-tert.butyl cinnamate, ethyl- alpha- acetyl-3, 5-dimethoxy- 4-hydroxy cinnamate, iso-propyl-alpha-acetyl-3,5-dimethoxy-4-hydroxy cinnamate, iso-amyl-alpha-acetyl-3,5-dimethoxy-4-hydroxy cinnamate, 2-ethylhexyl-alpha-acetyl-3,5-dimethoxy-4-hydroxy cinnamate, diethyl-3, 5-dimethoxy- 4-hydroxy benzylidene malonate, di-(2-ethylhexyl)-3,5-dimethoxy- 4-hydroxy benzylidene malonate, diisoamyl-3, 5-dimethoxy-4-hydroxy benzylidene malonate, dipalmitoyl-3, 5-dimethoxy-4-hydroxy benzylidene malonate, di-dodecyl-3,5-dimethoxy-4-hydroxy benzylidene malonate, and di-isopropyl-3,5-dimethoxy-4-hydroxy benzylidene malonate.

**15.** Use of a compound according to at least one of claims 1 to 14 to stabilize at least one additional sunscreen agent against degradation from exposure to light.

**16.** Use of a compound according to at least one of claims 1 to 14 to produce a a sunscreen formulation suitable to protect a substrate from UV radiation by applying said sunscreen formulation to said substrate.

**17.** A method of improving the photostability of a sunscreen formulation said method comprising adding a compound according to at least one of claims 1 to 14 to said sunscreen formulation in an amount sufficient to improve the photostability of said sunscreen agent, amount of compound added preferably to the sunscreen formulation falls within the range of 0.1 % to 40wt% of said sunscreen formulation.

**Patentansprüche**

**1.** Verbindung der Formel II

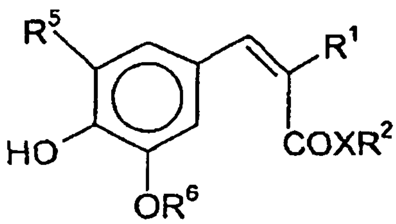

II

worin

$R^1$ aus der Gruppe bestehend aus $-C(O)CH_3$, $-CO_2R^3$, $-C(O)NH_2$ und $-C(O)N(R^4)_2$ ausgewählt ist;
X für O oder NH steht;
$R^2$ für lineares oder verzweigtes $C_1$- bis $C_{30}$-Alkyl steht;
$R^3$ für lineares oder verzweigtes $C_1$- bis $C_{20}$-Alkyl steht und
$R^4$ jeweils unabhängig voneinander für Wasserstoff oder lineares oder verzweigtes $C_1$- bis $C_8$-Alkyl steht;
$R^5$ für lineares oder verzweigtes $C_1$- bis $C_8$-Alkyl oder lineares oder verzweigtes $-O-C_{1-8}$-Alkyl steht und $R^6$ für

$C_1$- bis $C_8$-Alkyl steht.

**2.** Verbindung nach Anspruch 1, in der $R^6$ für $C_1$-$C_8$-Alkyl steht, X für Sauerstoff steht und $R^2$ für lineares oder verzweigtes $C_1$- bis $C_4$-Alkyl steht.

**3.** Verbindung nach mindestens einem der Ansprüche 1 oder 2, in der $R^1$ für $CO_2R^3$ steht und $R^3$ für lineares oder verzweigtes $C_1$- bis $C_8$-Alkyl, vorzugsweise $C_1$- bis $C_4$-Alkyl, steht.

**4.** Verbindung nach mindestens einem der Ansprüche 1 bis 3, in der $R^1$ für $C(O)CH_3$ steht.

**5.** Verbindung nach mindestens einem der Ansprüche 1 oder 2, in der $R^1$ für $C(O)N(R^4)_2$ steht und mindestens ein Rest $R^4$ für Wasserstoff und der andere für Wasserstoff oder lineares oder verzweigtes $C_1$- bis $C_4$-Alkyl steht.

**6.** Verbindung nach mindestens einem der Ansprüche 1 oder 2, in der $R^1$ für $C(O)N(R^4)_2$ steht und $R^4$ jeweils unabhängig voneinander für lineares oder verzweigtes $C_1$- bis $C_4$-Alkyl steht.

**7.** Verbindung nach Anspruch 1, in der $R^6$ für $C_1$-$C_4$-Alkyl steht, $R^1$ für $CO_2R^3$ steht und mindestens einer der Reste $R^2$ und $R^3$ für lineares oder verzweigtes $C_8$- bis $C_{20}$-Alkyl steht.

**8.** Verbindung nach Anspruch 7, in der $R^2$ und $R^3$ jeweils für lineares oder verzweigtes $C_8$- bis $C_{12}$-Alkyl stehen.

**9.** Verbindung nach Anspruch 7, in der mindestens einer der der Reste $R^2$ und $R^3$ für lineares oder verzweigtes $C_{12}$- bis $C_{20}$-Alkyl steht.

**10.** Verbindung nach mindestens einem der Ansprüche 1 bis 9, in der $R^6$ für Methyl oder Ethyl steht.

**11.** Verbindung nach Anspruch 1, bei der es sich um eine Verbindung der Formeln III oder IV

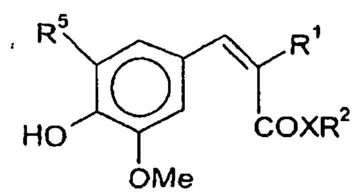

III

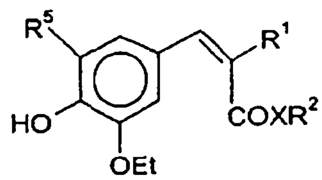

IV

worin

$R^1$ aus der Gruppe bestehend aus $-C(O)CH_3$, $-CO_2(C_1$-$C_8$-Alkyl), $-C(O)NH_2$ und $-C(O)N(C_1$-$C_4$-Alkyl$)_2$ ausgewählt ist;
X für O oder NH steht;
$R^2$ für $C_1$-$C_{12}$-Alkyl steht und
$R^5$ für lineares oder verzweigtes $C_1$-$C_8$-Alkyl oder lineares oder verzweigtes -O-$C_{1-8}$-Alkyl steht; handelt.

**12.** Verbindung nach Anspruch 11, in der X für Sauerstoff steht, $R^2$ für lineares oder verzweigtes $C_1$- bis $C_4$-Alkyl steht und $R^1$ für $-CO_2C_8H_{18}$ steht.

**13.** Verbindung nach mindestens einem der Ansprüche 1 bis 12, in der -O-$R^6$ und -$R^5$ identisch sind und vorzugsweise $R^5$ für -O-Methyl oder O-Ethyl steht und $R^6$ für -Methyl oder Ethyl steht.

**14.** Verbindung nach Anspruch 1, ausgewählt aus der Gruppe bestehend aus
Di-(2-ethylhexyl)-3-methoxy-4-hydroxy-5-isopropylbenzylidenmalonat,
Diisoamyl-3-methoxy-4-hydroxy-5-tert.-butylbenzylidenmalonat,
Isoamyl-alpha-acetyl-3-methoxy-4-hydroxy-5-isopropylcinnamat,
Isoamyl-alpha-acetyl-3-methoxy-4-hydroxy-5-tert.-butylcinnamat,
Ethyl-alpha-acetyl-3,5-dimethoxy-4-hydroxycinnamat,

Isopropyl-alpha-acetyl-3,5-dimethoxy-4-hydroxycinnamat,
Isoamyl-alpha-acetyl-3,5-dimethoxy-4-hydroxycinnamat,
2-Ethylhexyl-alpha-acetyl-3,5-dimethoxy-4-hydroxycinnamat,
Diethyl-3,5-dimethoxy-4-hydroxybenzylidenmalonat,
Di-(2-ethylhexyl)-3,5-dimethoxy-4-hydroxybenzylidenmalonat,
Diisoamyl-3,5-dimethoxy-4-hydroxybenzylidenmalonat,
Dipalmitoyl-3,5-dimethoxy-4-hydroxybenzylidenmalonat,
Didodecyl-3,5-dimethoxy-4-hydroxybenzylidenmalonat und
Diisopropyl-3,5-dimethoxy-4-hydroxybenzylidenmalonat.

**15.** Verwendung einer Verbindung nach mindestens einem der Ansprüche 1 bis 14 zur Stabilisierung mindestens eines zusätzlichen Sonnenschutzmittels gegen Abbau durch Einwirkung von Licht.

**16.** Verwendung einer Verbindung nach mindestens einem der Ansprüche 1 bis 14 zur Herstellung einer Sonnenschutzformulierung, die zum Schutz eines Substrats gegen UV-Strahlung durch Auftragen der Sonnenschutzformulierung auf das Substrat geeignet ist.

**17.** Verfahren zur Verbesserung der Lichtstabilität einer Sonnenschutzformulierung, bei dem man der Sonnenschutzformulierung eine Verbindung nach mindestens einem der Ansprüche 1 bis 14 in einer zur Verbesserung der Lichtstabilität des Sonnenschutzmittels ausreichenden Menge zugibt, wobei die der Sonnenschutzformulierung zugesetzte Menge an Verbindung vorzugsweise in den Bereich von 0,1 bis 40 Gew.-%, bezogen auf die Sonnenschutzformulierung, fällt.

## Revendications

**1.** Composé de formule II

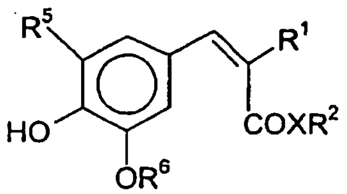

II

dans laquelle

$R^1$ est choisi dans le groupe constitué de $-C(O)CH_3$, $-CO_2R^3$, $-C(O)NH_2$ et $-C(O)N(R^4)_2$ ;
X est O ou NH ;
$R^2$ est un alkyle en $C_1$ à $C_{30}$ linéaire ou ramifié ;
$R^3$ est un alkyle en $C_1$ à $C_{20}$ linéaire ou ramifié ; et
chaque $R^4$ est indépendamment un hydrogène ou un alkyle en $C_1$ à $C_8$ linéaire ou ramifié,
$R^5$ est un alkyle en $C_1$ à $C_8$ linéaire ou ramifié ou -O-alkyle en $C_1$ à $C_8$ linéaire ou ramifié, et $R^6$ est un alkyle en $C_1$ à $C_8$.

**2.** Composé selon la revendication 1, dans lequel $R^6$ est un alkyle en $C_1$ à $C_8$, X est un oxygène et $R^2$ est un alkyle en $C_1$ à $C_4$ linéaire ou ramifié.

**3.** Composé selon au moins l'une des revendications 1 ou 2, dans lequel $R^1$ est $CO_2R^3$ et $R^3$ est un alkyle en $C_1$ à $C_8$ linéaire ou ramifié, de préférence alkyle en $C_1$ à $C_4$.

**4.** Procédé selon au moins l'une des revendications 1 à 3, dans lequel $R^1$ est $C(O)CH_3$.

**5.** Composé selon au moins l'une des revendications 1 ou 2, dans lequel $R^1$ est $-C(O)N(R^4)_2$, et au moins un $R^4$ est un hydrogène et l'autre est un hydrogène ou alkyle en $C_1$ à $C_4$ linéaire ou ramifié.

**6.** Composé selon au moins l'une des revendications 1 ou 2, dans lequel $R^1$ est $-C(O)N(R^4)_2$, et chaque $R^4$ est indépendamment un alkyle en $C_1$ à $C_4$ linéaire ou ramifié.

**7.** Composé selon la revendication 1, dans lequel $R^6$ est un alkyle en $C_1$ à $C_4$, $R^1$ est $-CO_2R^3$, et au moins l'un de $R^2$ et $R^3$ est un alkyle en $C_8$ à $C_{20}$, linéaire ou ramifié.

**8.** Composé selon la revendication 7, dans lequel $R^2$ et $R^3$ sont chacun un alkyle en $C_8$ à $C_{12}$ linéaire ou ramifié.

**9.** Composé selon la revendication 7, dans lequel au moins l'un de $R^2$ et $R^3$ est un alkyle en $C_{12}$ à $C_{20}$ linéaire ou ramifié.

**10.** Composé selon au moins l'une des revendications 1 à 9 dans lequel $R^6$ est un méthyle ou éthyle.

**11.** Composé selon la revendication 1, dans lequel le composé est de formule III ou IV

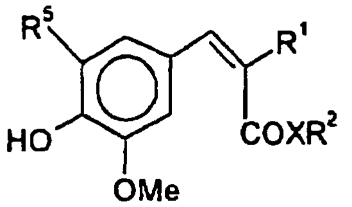 III 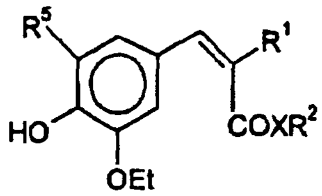 IV

dans laquelle

$R^1$ est choisi dans le groupe constitué de $-C(O)CH_3$, $-CO_2$(alkyle en $C_1$ à $C_8$), $-C(O)NH_2$ et $-C(O)N$(alkyle en $C_1$ à $C_4)_2$ ;
X est O ou NH ; et
$R^2$ est un alkyle en $C_1$ à $C_{12}$, et
$R^5$ est un alkyle en $C_1$ à $C_8$ linéaire ou ramifié ou -O-alkyle en $C_1$ à $C_8$ linéaire ou ramifié.

**12.** Composé selon la revendication 11, dans lequel X est un oxygène et $R^2$ est un alkyle en $C_1$ à $C_4$ linéaire ou ramifié et $R^1$ est $-CO_2C_8H_{18}$.

**13.** Composé selon au moins une des revendications 1 à 12, dans lequel $-O-R^6$ et $-R^5$ sont identiques, et de préférence $R^5$ est -O-méthyle ou -O-éthyle et $R^6$ est méthyle ou éthyle.

**14.** Composé selon la revendication 1 choisi dans le groupe constitué du
di-(2-éthylhexyl)-3-méthoxy-4-hydroxy-5-isopropylbenzylidène-malonate,
diisoamyl-3-méthoxy-4-hydroxy-5-tert.-butylbenzylidène-malonate,
isoamyl-α-acétyl-3-méthoxy-4-hydroxy-5-isopropylcinnamate,
isoamyl-α-acétyl-3-méthoxy-4-hydroxy-5-tert.-butylcinnamate,
éthyl-α-acétyl-3,5-diméthoxy-4-hydroxycinnamate,
isopropyl-α-acétyl-3,5-diméthoxy-4-hydroxycinnamate,
isoamyl-α-acétyl-3,5-diméthoxy-4-hydroxycinnamate,
2-éthylhexyl-α-acétyl-3,5-diméthoxy-4-hydroxycinnamate,
diéthyl-3,5-diméthoxy-4-hydroxybenylidène-malonate,
di-(2-éthylhexyl)-3,5-diméthoxy-4-hydroxybenzylidène-malonate,
diisoamyl-3,5-diméthoxy-4-hydroxybenzylidènemalonate,
dipalmitoyl-3,5-diméthoxy-4-hydroxybenzylidènemalonate,
didodécyl-3,5-diméthoxy-4-hydroxybenzylidènemalonate,
diisopropyl-3,5-diméthoxy-4-hydroxybenzylidènemalonate.

**15.** Utilisation d'un composé selon au moins l'une des revendications 1 à 14 pour stabiliser au moins un agent d'écran solaire additionnel contre une dégradation due à l'exposition à la lumière.

**16.** Utilisation d'un composé selon au moins l'une des revendications 1 à 14 pour produire une formulation d'écran solaire appropriée pour protéger un substrat du rayonnement UV en appliquant ladite formulation d'écran solaire

audit substrat.

**17.** Procédé d'amélioration de la photostabilité d'une formulation d'écran solaire, ledit procédé comprenant l'addition d'un composé selon au moins l'une des revendications 1 à 14 à ladite formulation d'écran solaire en une quantité suffisante pour améliorer la photostabilité dudit agent de protection solaire, la quantité de composé ajouté de préférence à la formulation d'écran solaire se trouve dans la gamme de 0,1 % à 40 % en poids de ladite formulation d'écran solaire.